Europäisches Patentamt

European Patent Office

Office européen des brevets

(11) Veröffentlichungsnummer: **0 283 873**
**A2**

(12) **EUROPÄISCHE PATENTANMELDUNG**

(21) Anmeldenummer: **88103855.8**

(22) Anmeldetag: **11.03.88**

(51) Int. Cl.⁴ **A61F 13/00**

(30) Priorität: **27.03.87 DE 3710115**
**03.10.87 DE 3733502**
**04.02.88 DE 8801358 U**
**07.01.88 DE 8800091 U**
**18.02.88 DE 8802059 U**

(43) Veröffentlichungstag der Anmeldung:
**28.09.88 Patentblatt 88/39**

(84) Benannte Vertragsstaaten:
**AT BE CH DE ES FR GB GR IT LI LU NL SE**

(71) Anmelder: **Budde, Franz-Josef**
**Badestrasse 53**
**D-4714 Selm(DE)**

(72) Erfinder: **Budde, Franz-Josef**
**Badestrasse 53**
**D-4714 Selm(DE)**

(74) Vertreter: **Herrmann-Trentepohi, Werner,**
**Dipl.-Ing. et al**
**Schaeferstrasse 18**
**D-4690 Herne 1(DE)**

(54) **Medizinische Applikation zur Bekämpfung von Reizzuständen, insbesondere der Muskulatur, der Sehnen und der Gelenke.**

(57) Bei einer Bandage anzulegen bei Reizzuständen der Muskulatur, der Sehnen und der Gelenke, wie z. B. Epicondylitis oder rheumatischen Beschwerden, bestehend aus einem auf die behandelnde Körperstelle aufzulegenden Bandagenkörper und einseitig daran befestigten Bändern zur Fixierung des Bandagenkörpers ist erfindungsgemäß vorgesehen, daß der Bandagenkörper schichtweise aufgebaut ist und aus einer Lage aus einem Baumwollgewebe und einer Lage aus einem Kunstfasergewebe besteht, zwischen denen eine weitere Lage aus offenzelligen Schaumstoff angeordnet ist, welcher ein Thermovlies bildet.

Fig. 1

Die Erfindung betrifft eine medizinische Applikation zur Bekämpfung von Reizzuständen, insbesondere der Muskulatur, der Sehnen und der Gelenke gemäß dem Oberbegriff des Anspruchs 1.

Die erfindungsgemäße Applikation wird äußerlich angewandt und dient z. B. zur Behandlung des rheumatischen Formenkreises, wobei es sich um Gelenkschmerzen oder auch um den bekannten Hexenschuß handeln kann. Ein anderes Anwendungsbeispiel der Erfindung ist die Behandlung der Epicondylitis, unter der häufig Handwerker oder Sportler leiden, unter denen allgemein unter dem Namen Tennisarm bekannt ist.

Zur Behandlung speziell der Epicondylitis ist u. a. eine aus Kunststoff hergestellte Spange bekannt, die im Querschnitt U-förmig ausgebildet ist. Die U-Schenkel legen sich auf die gereizten Sehnen. Der die Schenkel verbindende U-Bogen dient als vorgespanntes Federelement und verteilt den Druck der Schenkel auf die Sehnen. Dieses rein mechanische Hilfsmittel dient nur zur Entlastung der Unterarmmuskulatur. Soweit rheumatische Beschwerden eine Wärmeumwandlung auf der erkrankten Körperstelle voraussehen, sind derartige Spangen nicht brauchbar. Technische Schwierigkeiten bereiten auch Spangen, die z. B. im Nacken oder Rückenbereich angesetzt werden müssen.

Es werden daher Materialien verwendet, die einen Wärmespeichereffekt besitzen. Darunter fallen Röhren aus Thermopren, die ein zu behandelndes Knie, einen Ellenbogen oder auch den Lendenbereich umgeben. Zur Gewährleistung der Bewegungsfreiheit werden elastische Kunstfasern bzw. Kunststoffe eingesetzt. Diese führen jedoch die vom Körper abgegebene Feuchtigkeit nicht ab und lösen dadurch im Laufe der Zeit unangenehmen Juckreiz bzw. auch Infektionen aus. Da die Extremitäten gänzlich eingeschlossen werden, kann es zur Einschränkung der Blutzirkulation kommen.

Der Erfindung liegt die Aufgabe zugrunde, eine medizinische Applikation der eingangs genannten Art so auszubilden, daß sie keine schädlichen Nebenwirkungen auslösen kann, jedoch eine Wärmeanwendung ausreichender Kapazität zur Förderung der Blutzirkulation gestattet.

Die Erfindung löst diese Aufgabe durch die Merkmale des kennzeichnenden Teils des Anspruchs 1. Weitere Merkmale der Erfindung sind Gegenstand der Unteransprüche.

Durch den schichtweisen Aufbau des Bandagenkörpers entsteht ein voluminöses Gebilde, dessen Füllung das Thermovlies aus offenzelligem Schaumstoff ist. Dieses Material hat eine sehr große Wärmespeicherkapazität und sorgt für eine gleichmäßige Verteilung der Wärme. Die dem Körper abgewandte Seite der Bandage besteht dagegen aus einem schlechten Wärmeleiter, nämlich einem dichten Kunstfasergewebe, so daß die im Thermovlies eingespeicherte Wärme nicht nach außen entweichen kann. Die auf der zu behandelnden Körperstelle aufliegende Lage der Bandage besteht hingegen aus einem Wollgewebe natürlicher Provinienz z. B. aus Baumwolle und gibt die von der Körperstelle stammende Wärme an das Thermovlies weiter. Sie hat darüberhinaus den Vorteil, die von der Körperfläche abgegebene Feuchtigkeit aufzunehmen. Das wirkt sich auf den Heilungsprozeß zusätzlich positiv aus. Die Wärmeverteilung des Thermovlieses beruht darauf, daß es aus vielen Einzelfasern besteht und seine Offenzelligkeit die Zirkulation der Wärme gewährleistet. Die erfindungsgemäße Anordnung der Schichten be. wirkt eine Hauttemperaturerhöhung von ca. 8 Grad nach nur 15 bis 30 Minuten. Das zeigen Untersuchungen, welche demonstrieren, daß eine optimale Durchblutung in der gleichen Zeit erfolgt die zu behandelnde Körperstelle langsam und auf natürliche Weise um ca. 8 Grad erwärmt wird, erweitern sich die Kapilaren des Unterhautbindegewebes mit der Folge eines vermehrten Flüssigkeitsaustausches zwischen Blut und Gewebe.

Die eingebauten Magnetfolien dienen zur Verbesserung und Stabilisierung der beschriebenen und die Therapie bewirkenden Effekte. Sie erzeugen Magnetfelder, die im Zusammenwirken mit der Wärme einen gesteigerten Sauerstoffpartialdruck, eine Polarisierung der Membranen in den Körperzellen und dadurch bedingt eine Verbesserung des Energiestoffwechsels im Organismus bewirken. Damit werden die körpereigenen Abwehrkräfte verstärkt und der Selbstheilungsprozeß beschleunigt.

Zweckmäßigerweise sind die Magnetfolien in der Nähe der zu behandelnden Körperstelle angeordnet. Das ist Gegenstand des Anspruches 2.

Als Kunstfasergewebe, das ein Entweichen der in dem Thermovlies gespeicherten Wärme verhindert, ist besonders geeignet das Merkmal des Anspruches 3.

Mit den Ansprüchen 4 und 5 werden die Bänder weitergebildet. Ihre Elastizität der Bänder ermöglicht die Anpassung der Bandage an die verschiedensten Körpergrößen. Der Klettverschluß ergibt eine einfache Fixierung und ist besonders angenehm, wenn die erkrankte Person durch die Reizung in ihrer Bewegungsfähigkeit eingeschränkt ist, so daß ihr ein Schließen von Schnallen oder Knöpfen nur mit großen Schwierigkeiten möglich ist.

Die Magnetfolien sind in rechteckige Ausschnitte, welche linearmagnetisiert und in parallelen Reihen angeordnet sind, welche regelmäßige Abstände der parallelen der Folien aufweisen. Es hat sich jedoch überraschend herausgestellt, daß man auf einfache Weise eine verbesserte Wirkung der Magnete erreichen kann, wenn man diesen Verlauf der Magnete ändert. Das ist Inhalt des Anspruches 6.

Die zirkulare Anordnung der Magnete beeinflußt die von den Feldern erfaßten Blutbahnen in der Weise, daß im Ergebnis eine Verwirbelung der auf die Magnete ansprechenden Blutkörper in der Blutsubstanz bzw. dem Gewebe eintritt. Die Magnete liefern die hierfür erforderliche Energie, die in Wärme umgesetzt wird, welche den therapeutischen Effekt der Bandage merklich verbessert. Vorzugsweise verwirklicht man die Erfindung mit den Merkmalen des Patentanspruches 7. Damit erzielt man nämlich eine weitgehende Reihenschaltung der Magnete und damit die erforderlichen Feldstärken.

Die Erfindung hat insoweit den Vorteil, daß sie auf einfache Weise die Wirkungen der Magnete verstärkt, insbesondere die Blutzirkulation in den befallenen Körperregionen anregt und neuartige Wirkungen erzielt, wobei die erzeugte Wärme für den gewünschten therapeutischen Effekt nutzbar ist.

Je nach der Ausdehnung und der Form der befallenen Körperstellen läßt sich die beschriebene Wirkung der Erfindung verbessern, wenn man die Erfindung mit den Merkmalen des Patentanspruches 8 verwirklicht. Während die kreisscheibenförmigen Magnete lediglich einfach zirkularmagnetisiert sind, d. h. die konzentrischen Ringe parallel zu der Umrißlinie der Kreisscheibe verlaufen, gelingt es, mit den rechteckigen bis quadratischen Magnetfeldlinien mehrere Zirkularmagnetisierungen eng aneinander anzuschliessen und dadurch die Wirkung erheblich auf ausgedehntere Körperregionen zu konzentrieren.

Die Magnetfolie selbst ist notwendigerweise ein mehrschichtiges Gebilde, in das die magnetisierbaren und die magnetbildenden Eisenteile eingeschlossen sind. Diese Folie ist in gewissem Maße flexibel, läßt sich aber nicht über die volle Fläche der Bandage ausdehnen, wenn u. a. die Anpassungsfähigkeit an die befallenden Körperteile und die Belastung durch das Gewicht der Bandage optimal gehalten werden sollen. Mit den Merkmalen des Patentanspruches 9 gelingt es jedoch, das magnetische Feld auszudehnen, weil die Nickelfolie ihrerseits magnetisiert wird und andere nützliche Eigenschaften aufweist.

Die Erfindung bezieht sich ferner auf die Bekämpfung von Migränezuständen.

Bei der Migräne handelt es sich um anfallsweise, oft einseitig auftretende Kopfschmerzen, die gewöhnlich stundenlang anhalten, meist mit Übelkeit, Erbrechen und Überempfindlichkeit der Sinnesorgane verbunden sind und zu periodischer Wiederkehr neigen. Eine häufige Begleiterscheinung ist das bekannte Flimmerskotum. Neben solchen flüchtigen Sehstörungen kann es auch zu bereichsweisen motorischen Lähmungen, asphasischen u. a. zerebralen Herderscheinungen kommen. Bei schweren Auftritten sind Bewußtseinsänderungen bis zu Migränedämmerzuständen nicht selten. Als Ursache wird heute eine abnorme Krampfbereitschaft der Hirngefäße angenommen, der wiederum konstituionelle Momente, allergische Überempfindlichkeit und sonstige humorale Besonderheiten zugrundeliegen. Mannigfache exogene Momente, wie Zyklusstörungen bei Frauen, Magen-, Darm-Affektionen, psychische Erregungen kommen als auslösende Momente des jeweiligen Anfalls in Frage. Weitere Ursachen können neuralgiforme, unter mechanischen Einflüssen momentan auftretende anfallartige Schmerzen im Nacken-und Hinterkopf mit Ausstrahlung nach Schläfe und Stirn sein, die häufig mit Ohrgeräuschen und Schwindelgefühl verbunden sind. Die Beschwerden werden in Zusammenhang gebracht mit einer auf Bandscheibenschädigung beruhenden, vorwiegend einseitigen sogenannten spondylotischen Deformierung der Halswirbelkörper infolge asymmetrischer Belastung unter Mitwirkung neuraler oder vaskulärer Momente. Zur Behandlung der Migräne werden in verstärktem Maße Medikamente eingesetzt, die bei dauernder Einnahme zu organischen Schäden sowie zur Abhängigkeit führen können.

Die Erfindung schafft ein Hilfsmittel zu Linderung bzw. Beseitigung der bei Migräneanfällen auftretenden Kopfschmerzen mit den Merkmalen des Anspruchs 17.

Der Streifen aus flexibler Magnetfolie ist axial magnetisiert. D. h., daß eine Streifenoberfläche den magnetischen Südpol und die entgegengesetzt liegende Oberfläche den magnetischen Nordpol trägt. Wichtig bei dem erfindungsgemäßen Stirnband ist, daß der magnetische Nordpol zum Kopf des Stirnbandträgers weist. Die Länge des Magnetstreifens ist dabei so bemessen, daß er im angelegten Zustand die gesamte Stirnpartie und die Schläfenpartien bis mindestens zum Ohrbereich bedeckt. Somit wird in diesem Bereich gezielt ein Magnetfeld aufgebaut, das infolge der Axialmagnetisierung eine große Eindringtiefe in den Kopf des Stirnbandträgers aufweist. Durch das Magnetfeld wird die Durchblutung der kapillaren Endstrombahnen und damit die O2-Utilisation verbessert. Das Magnetfeld wirkt auf jede Körperzelle im Stirn-und Schläfenbereich, in dem dort das Zellgrenzmembranpotential signifikant erhöht wird. So kommt es zu einer Verbesserung der Zellatmung und damit des gesamten Zellstoffwechsels, welcher ohnehin bei den chronischen Erkrankungen deutlich gesenkt ist. Aber auch die humoralen und hormonalen dienervalen und kolloidalen Systeme werden in positiver Weise beeinflußt. Es wird vermutet, daß die magnetischen Felder im Organismus die Aktivierung bioenergetischer Energie bewirken, indem dort die sogenannte "Homöostase", d. h. das stabile Gleichgewicht wieder hergestellt wird. Insgesamt werden durch das angelegte Magnetfeld bioenergetische Abläufe im Organismus aktiviert, optimiert und reorganisiert.

Es hat sich herausgestellt, daß die besten Ergebnisse erzielt werden, wenn die Magnetfeldstärke ca. 290 Gaus beträgt. Bei empfindlichen Patienten kann jedoch auch schon eine Magnetfeldstärke von ungefähr 150 Gaus ausreichend sein.

Um das Stirnband unterschiedlichen Kopfgrößen anpassen zu können und bei unterschiedlich großen Köpfen einen sicheren Sitz zu gewährleisten, sind die beiden Enden des Magnetstreifens durch ein längenveränderliches Band miteinander verbunden. Es kann sich dabei um ein unelastisches Band handeln, das mit Hilfe eines Klettverschlusses oder einer Schnalle das Stirnband am Kopf fixiert.

Vorteilhafterweise besteht das längenveränderliche Band aber aus einem elastischen Gewebe. Dieses elastische Gewebe paßt sich automatisch der jeweiligen Kopfform an, ohne daß störende Verschlüsse im Hinterkopfbereich nötig sind.

Gemäß dem Merkmal des Anspruchs 19 ist der Stoffüberzug ein Frotteegewebe aus hochwertigen Naturringarnen. Diese Naturgarne sind besonders hautfreundlich und weisen eine hohe Aufnahmefähigkeit für Feuchtigkeit auf. Diese Aufnahmefähigkeit wird noch dadurch gesteigert, daß es sich bei dem Gewebe um ein Frotteegewebe handelt. Ein derartiger Stoffüberzug ist im Hinblick auf die immer wieder auftretenden Schweißausbrüche bei Migräneanfällen besonders vorteilhaft. Darüberhinaus hat der Frotteeüberzug eine gewisse polsternde Wirkung, so daß auch längeres Tragen des Stirnbandes problemlos ist.

Das Merkmal des Anspruchs 20 sieht vor, daß der Magnetstreifen zumindest stellenweise zirkularmagnetisiert ist. Unter Zirkularmagnetisierung versteht man dabei eine Magnetfeldkonfiguration, bei der in einer Ausführungsform einer zentralen Magnetisierung ringförmig diese umgebend Magnetisierungen mit der entgegengesetzten Polarisierung folgen, wobei benachbarte Ringe jeweils ungleichnamige Pole aufweisen. Bei einem anderen Ausführungsbeispiel wird die Zirkularmagnetisierung dadurch hergestellt, daß um einen Mittelpunkt herum segmentförmige Magnetisierungen angeordnet sind, wobei die Segmente Tortenstücken ähneln. Dabei weisen jeweils benachbarte Segmente ungleichnamige Polarisation auf. Die Zirkularmagnetisierung bewirkt eine Verwirbelung der Magnetfeldlinien. Durch die Verwirbelung haben die Magnetfeldlinien zwar nicht eine so große Eindringtiefe, wie die Magnetfeldlinien der Axialmagnetisierung, sie verstärken jedoch deren Wirkung im Hirnschalenbereich.

Die Erfindung ist ferner auf eine Decke anwendbar, welche insbesondere als Unterbett dient, wobei die Liegefläche aus Schafwolle besteht.

Die wohltuende und beruhigende Wirkung derartiger Schafwolldecken ist seit langem bekannt. Sie werden als Webdecke oder als Fell angeboten. Felle werden insbesondere für Kleinkinder und Säuglinge empfohlen, die einen unruhigen Nachtschlaf haben. Nachteilig bei derartigen Decken ist es, daß durch die anhaltende Belastung der Flausch bzw. das Fell der Decken zusammengepreßt wird, so daß der anfängliche gute Temperaturausgleich durch die lockerstehenden Wollfasern mit der Zeit immer schlechter wird. Somit ist nach absehbarer Zeit die Wirkung der Decke verlorengegangen, im Winter warmzuhalten und im Sommer den schweißtreibenden Wärmestau zu verhindern.

Gemäß der Erfindung wird die Decke der genannten Art so ausgebildet, daß sie ihre temperaturausgleichende Wirkung über einen langen Zeitraum beibehält und das Wohlbefinden des auf der Decke Ruhenden noch steigert.

Das wird erfindungsgemäß dadurch erreicht, daß zwischen der Schafwollage und einer zweiten Schicht aus Baumwollgewebe eine Schicht aus Polyester-Vlies vorgesehen ist, welches aus einem offenzelligen Schaumstoff gebildet ist, und in welchem folienförmige, flexible Magnetelemente eingebettet sind. Durch die besondere Materialwahl und den schicht weisen Aufbau dieser Materialien, nämlich z. B. Merinoschafwolle, locker aufgebautes Faservlies und 100 % atmungsaktive Baumwolle, wird eine Decke geschaffen, die ihre wärmeausgleichende Wirkung praktisch nicht verliert, da sich das Polyestervlies nach jeder Belastung wieder aufrichtet. Durch die Pufferwirkung des Polyester-Vlieses werden auch die Schafwollfasern nicht so fest aufeinandergepreßt wie bei den bekannten Decken. Somit steigert die erfindungsgemäße Decke allein schon durch ihren Aufbau und die Materialwahl das Wohlbefinden des Schlafenden. Durch den Einsatz der Magnetelemente in der Decke kann die wohltuende und beruhigende Wirkung der Decke noch gesteigert werden. So wird seit langem vermutet, und die Forschungen der letzten Zeit scheinen dies zu bestätigen, daß insbesondere das erdmagnetische Feld gerade auf das Wohlbefinden des Menschen einen großen Einfluß hat. Das erdmagnetische Feld kann jedoch durch z. B. Wasseradern oder größere Metallagerstätten so gestört sein, daß empfindsame Menschen auf diese Störung negativ reagieren. Durch den Einsatz der Magnetelemente, deren Feldstärke etwa viermal so groß ist wie das normale Erdmagnetfeld, werden die Erdmagnetfeldstörungen ausgeschaltet, da sie im Verhältnis zum künstlich erzeugten Magnetfeld vernachlässigbar klein sind.

Gerade in modernen Hochhäusern ist die Abschirmung gegen das Erdmagnetfeld z. T. so groß, daß innerhalb des Gebäudes ein nur noch geringes oder gar kein Magnetfeld wirkt. Die in der Decke angeordneten Magnete stellen somit ein simulierendes und stärkeres Erdmagnetfeld dar.

Wie die Natur lehrt, die Schlafgewohnheiten wilder Tiere deuten das an, ist die beste Schlaflage in nord-südlicher Richtung. Daher schlägt der Anspruch 2 vor, die Magnetelemente in der Decke so anzuordnen, daß die im kopfendseitigen Bereich der Decke angeordneten Magnetfolien mit ihren magnetischen Südpolen zum Körper des Liegenden und die im fußendseitigen Bereich der Decke angeordneten Magnetfolien mit ihren Nordpolen zum Körper des Liegenden weisen. Somit ahmt die Decke ein natürliches Magnetfeld in geografischer Nord-Süd-Richtung nach, wobei jedoch die Feldstärke größer ist als beim natürlichen Magnetfeld.

Nach den Merkmalen des Anspruchs 3 sind in den kopfendseitigen Magnetfolien und zwar im mittleren Bereich der Decke der axialen Magnetisierung Zirkularmagnetisierungen aufgeprägt. Diese zusätzlichen Zirkularmagnetisierungen haben eine starke Tiefenwirkung, die die wohltuende und aktivierende Wirkung der Decke noch verstärken, speziell bei Verspannungen im Nacken-und Rückenbereich. Diese an sich aus der US-PS 45 49 532 bekannte Zirkularmagnetisierung wird durch Magnete erzeugt, die in konzentrischen Ringen mit jeweils abwechselnder Polarität angeordnet sind.

Überraschenderweise hat sich herausgestellt, daß mit der erfindungsgemäßen Decke die besten Ergebnisse erzielt werden, wenn die Magnetelemente in der Grenzschicht des Polyester-Vlieses zur Schlafwolleinlage angeordnet sind, d. h. möglichst nahe am Körper der ruhenden Person.

Zur Erhöhung der Strapazierfähigkeit der Decke und damit zur Verlängerung ihrer Lebensdauer schlägt der Anspruch 5 vor, die Oberfläche der Polyester-Vliesschicht mit einer dünnen Harzschicht zu versehen. Diese dünne Harzschicht hat darüberhinaus die Fähigkeit, das ohnehin schon hohe Isolationsvermögen des Polyester-Vlieses noch zu erhöhen.

Damit sich die Wärme in der Polyester-Vliesschicht gut und gleichmäßig verteilt, ist das Polyestervlies gemäß Anspruch 6 aerodynamisch geblasen.

Die Erfindung ist schließlich auch für veterinärmedizinische Applikationen zur Wärmebehandlung isolierter Körperpartien gemäß dem Oberbegriff des Anspruches 23 nützlich.

Solche Applikationen werden, wenn auch nicht ausschließlich, so doch bislang hauptsächlich auf Pferde angewandt. Hierbei zieht man die Wirbelsäule, die Lende und die Kruppe, aber auch die Sprung-oder Karpalgelenke als Körperpartien des Tieres in Betracht, die mit dem erfindungsgemäßen Umschlag behandelt werden. Der Form dieser und anderer Körperpartien ist der Umriß des Umschlages angepaßt. Für die anderen Körperpartien genügt häufig eine Decke mit einem oder mehreren Bauchgurten, während zum Bandagieren der Gelenke Gamaschen verwendet werden. Der therapeutisch bzw. profilaktisch wirksame Teil der erfindungsgemäßen Bandage besteht jedoch aus einem Kissen, von dem mehrere in Betracht kommen, wenn von dem Umschlag mehr als eine Körperpartie erfaßt wird. Das gilt für die erwähnten Decken, die zur Unterbringung der erforderlichen Kissen entsprechende Taschen aufweisen.

Die Kissen der erfindungsgemäßen Bandage sollen die Temperatur der bandagierten Körperpartien des Tieres steigern, indem sie diese Körperpartien nach außen isolieren und dadurch einen Wärmestau hervorrufen. In der Bandage nimmt der der Haut zugekehrte Streifen des Bezuges auch Feuchtigkeit auf, welche zuammen mit der gespeicherten Wärme den Behandlungs effekt begründet. Dieser besteht aber auch im wesentlichen Umfang aus der Wirkung, die von dem Magnetstreifen ausgeht. Die Magnetisierung ergibt in der Bandage ein Energiepotential, das in der betreffenden Körperpartie freigesetzt wird. In dieser kommt es dadurch zu einem aktivierten Muskelstoffwechsel, der einerseits die beschleunigte Abführung von schädlichen Stoffwechselprodukten bewirkt und andererseits den Sauerstoffgehalt des Muskel-und des Bindegewebes steigert, wodurch der Behandlungserfolg wesentlich begünstigt wird.

Erfindungsgemäße veterinärmedizinische Applikationen ünterstützen deswegen die Behandlung bei Erkältung und Lungenentzündung, wirken vorbeugend bei Kreuzverschlag, Schulterlahmheit und Gelenkerkrankung, unterstützen auch auch die jeweilige Therapie. Entsprechendes gilt für die Gelenkbehandlung.

Die hierfür hauptsächlich verantwortlichen Kissen passen sich in ihrer flachen Form der betreffenden Körperpartie an. Wenn die Bandage auf Gelenke angewandt wird, kann das Kissen mit quer zur Wickelrichtung der Bandage verlaufenden Querkanten absteppt und damit noch anpassungsfähiger sein. Je geringer der Umfang der zu behandelnden Körperpartie ist, desto höhere Anforderungen werden an die Flexibilität des Kissens gestellt, um den vollflächigen Hautkontakt sicherzustellen. Je geringer der Spalt zwischen der Haut und dem Magnetstreifen ist, desto höher muß die Energiedichte der Magnetstreifen sein, um die gewünschte Wirkung sicherzustellen. Allgemein gesehen, sollte deshalb die Energiedichte möglichst hoch sein, um die geschilderten therapeutischen bzw. profilaktischen Wirkungen zu erreichen.

Es ist bekannt, Pferdedecken als Applikation mit Taschen für die Kissen zu versehen und Gamschen so auszubliden, daß ein Kissen der beschriebenen Art unter dem Wickel und unmittelbar auf der Haut angeordnet werden kann. Hierbei sind die Magnetstreifen in der Mitte des Kissens in mehreren parallelen Querreihen und längs der Diagonalen angeordnet. In jeder Reihe oder Diagonale sind mehrere Stabmagnete miteinander ausgefluchtet. Die dazwischenliegenden magnetfreien Räume stellen die notwendige Flexibilität her, führen aber auch dazu, daß sich die Magnetisierung auf relativ schwache Felder beschränkt, die sich in den vergleichsweise großen Abständen zwischen den ungleichnamigen Magnetpolen der in den Reihen angeordneten Magnetstreifen ausbilden. Daraus ergibt sich eine relativ gerinfe Energiedichte, welche im Ergebnis die mit der Magnetisierung beabsichtigten therapeutischen bzw. profilaktischen Wirkungen unzureichend erscheinen lassen.

Demgegenüber erlaubt es die Erfindung, die Energiedichte auf vorgegebenen Körperpartien zu steigern, ohne daß dadurch die Anpassungsfähigkeit des Kissens an die Bandage verringert wird. Das gelingt hauptsächlich mit den Merkmalen des Anspruches 23.

Die Anordnung der Magnetelemente in Streifen einer Folie nutzt die Flexibilität von Kunststoffolien geringer Wandstärke und Breite, weshalb einerseits die Anpassung der Bandage auch bei geringem Wickelradius nicht beeinträchtigt ist, andererseits aber auch die Magnetisierung auf der gesamten Streifenlänge unmittelbar aneinander anschließender Magnetpole schafft, was eine Erhöhung der Energiedichte in den Folien zwischen ungleichnamigen Polen ergibt. Die Strahlenanordnung gleichnamiger Magnetpole gemäß der Erfindung jeweils um einen gemeinsamen Mittelpunkt führt zur Ausbildung von tangential und radial angeordneten Magnetfeldern. Diese Anordnung induziert eine entsprechende Bewegung in den Elementarmagneten des behandelten Körperteiles, die von dem magnetischen Feld der Bandagenmagnete beeinflußt werden. Bei der Erfindung ist die Wirkung der Magnetfelder auf den betreffenden Körperteil entsprechend gesteigert, wodurch auch tiefere Schichten des Gewebes günstig beeinflußt werden.

Der Vorteil der erfindungsgemäßen Applikation liegt vor allem darin, daß sich auf dem Kissen erheblich mehr Energie in Form von Magnetfeldern unterbringen läßt, und daß sich die hohe Energiedichte in einer vergrößerten Feldstärke auswirkt, die ihrerseits den mit der Behandlung beabsichtigten Zweck verbessert. Deswegen läßt sich die Anzahl der Magnetstreifen auf wenige Folienabschnitte reduzieren, was die Bandagierung wesentlich erleichtert.

Vorzugsweise und mit den Merkmalen des Anspruches 24 wird die Magnetisierung so durchgeführt, daß sich die Energie auf eine oder auf wenige, um begrenzte Bereich begrenzte Flächen konzentriert. Bei der beanspruchten Flügelanordnung sind die Schrauben-und die Nabenfläche gleichmäßig, wodurch die Flächen zwischen den Schraubenflügeln die Gegenpole aufweisen. Die Magnetfelder verlaufen deswegen tangential um die Nabe, aber auch radial zur Nabe, wodurch den Elementarmagneten des behandelten Körperteiles eine wirbelnde Bewegungsresultierende aufgeprägt wird, sobald sie in die derart angeordneten Magnetfeldanordnungen des Folienstreifens geraten. Die Merkmale des Anspruches 25 ergeben eine optimale geometrische Folienausbildung in Bezug auf die begrenzte Breite des Folienabschnittes, die ihrerseits zur Erleichterung der Bandagierung gewünscht wird. In dem im Anspruch 26 beschriebenen Detail wird diese Anordnung im dem beschriebenen Sinne optimiert.

Besonders vorteilhaft wirkt sich die Erfindung auf die quer zur Wickelrichtung der Bandage abgesteppten Kissen aus. Hierbei ist nämlich die Diagonalanordnung nicht mehr ausführbar, was in den bekannten Applikationen dazu führt, daß die Energiedichte noch erheblich verringert wird. Mit den Merkmalen des Anspruches 27 läßt sich aber eine hohe Energiedichte auch bei quer zur Wickelrichtung der Bandage abgesteppten Kissenabschnitten erreichen.

Die Einzelheiten der Erfindung ergeben sich aus der nachfolgenden Beschreibung einer Ausführungsform anhand der Figuren in der Zeichnung; es zeigen

Fig. 1 in Draufsicht und Seitenansicht eine Bandage anzulegen bei Epicondylitis,

Fig. 1a alternative Ausführungsform der Bandage gemäß Fig. 1,

Fig. 2 in Draufsicht und Seitenansicht eine Schultergelenksbandage,

Fig. 3 in Draufsicht und Seitenansicht eine Schulter-/Nackenbandage,

Fig. 4 in Draufsicht und Seitenansicht eine Rückenbandage,

Fig. 5 in Draufsicht und Seitenansicht eine Bandage für den Ellenbogen, das Knie und das Knöchelgelenk,

Fig. 6 in perspektivischer Darstellung einen Ausschnitt aus einer Bandage gemäß der Erfindung,

Fig. 7 eine rechteckige Magnetfolie in Draufsicht unter Wiedergabe der Magnetisierungslinien gemäß einer ersten Ausführungsform der Erfindung,

Fig. 8 in der Fig. 7 entsprechender Darstellung eine andere Ausführungsform der Erfindung,

Fig. 9 eine kreisscheibenförmige Magnetfolie gemäß der Erfindung,

Fig. 10 in Draufsicht und in abgebrochener Darstellung einen erfindungsgemäßen Zuschnitt einer medizinischen Bandage für ein mensch liches Schultergelenk in Draufsicht und in abgebrochener Darstellung,

Fig. 11 in der Fig. 10 entsprechender Darstellung den Zuschnitt einer Bandage für den menschlichen Schulter-Nackenbereich,

Fig. 12 den Zuschnitt einer erfindungsgemäßen Bandage für den Rücken-bzw. Lendenbereich in den Fig. 10 und 11 entsprechender Darstellung,

Fig. 13 einen Zuschnitt einer erfindungsgemäßen Bandage für den menschlichen Ellenbogen, die auch für den Knie-oder Knöchelbereich des Menschen verwendbar ist in den Fig. 10 bis 12 entsprechender Darstellung,

Fig. 14 den Zuschnitt einer den Fig. 1 bzw. 1 a entsprechenden Armbandage,

Fig. 15 ein Stirnband gemäß der Erfindung,

Fig. 16 einen nicht maßstäblichen Querschnitt einer erfindungsgemäßen Decke,

Fig. 17 eine Draufsicht der Decke mit angedeuteten Magnetelementen,

Fig. 18 eine schematische Darstellung der Decke im Längsschnitt,

Fig. 19 ein Magnetelement in abgebrochener Darstellung mit aufgeprägter Zirkularmagnetisierung,

Fig. 20 eine Prinzipdarstellung einer Zirkularmagnetisierung,

Fig. 21 eine abgewandelte Anorndung der Zirkularmagnetisierung gemäß Fig. 4,

Fig. 22 in Draufsicht eine Ausführungsform einer erfindungsgemäßen veterinärmedizinischen Applikation, von der jedoch nur das Kissen wiedergegeben ist,

Fig. 23 einen Teilschnitt durch den Gegenstand der Fig. 22 und

Fig. 24 die Wiedergabe eines Folienabschnittes mit seiner Magnetisierung.

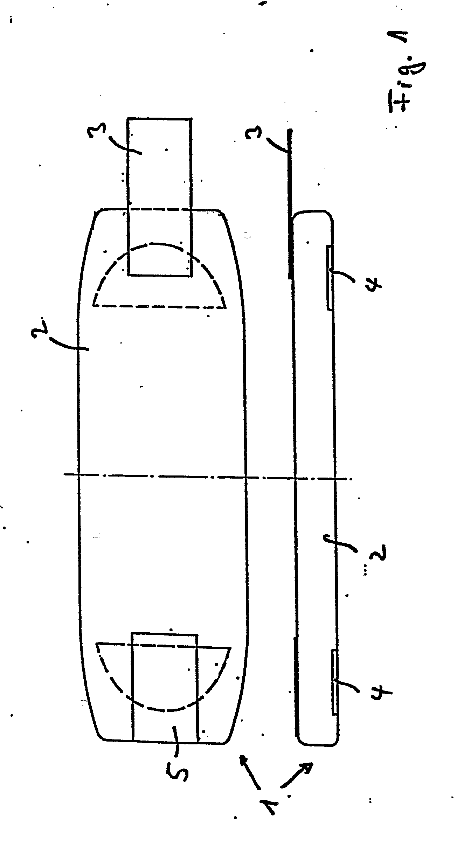

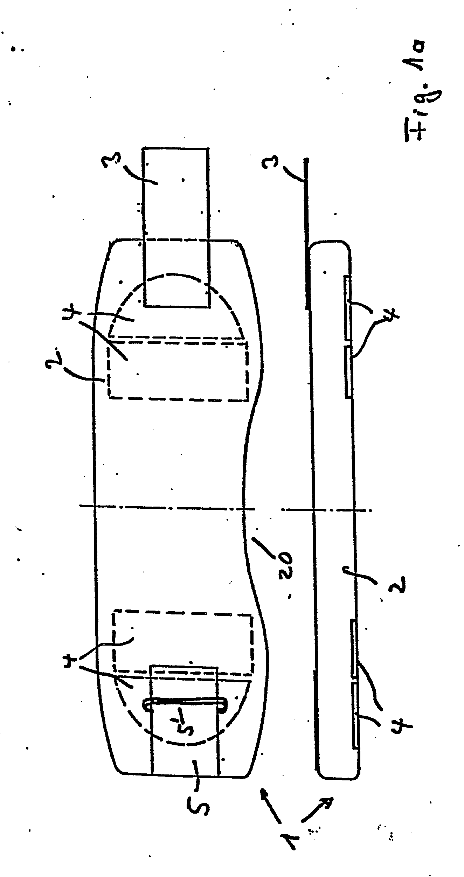

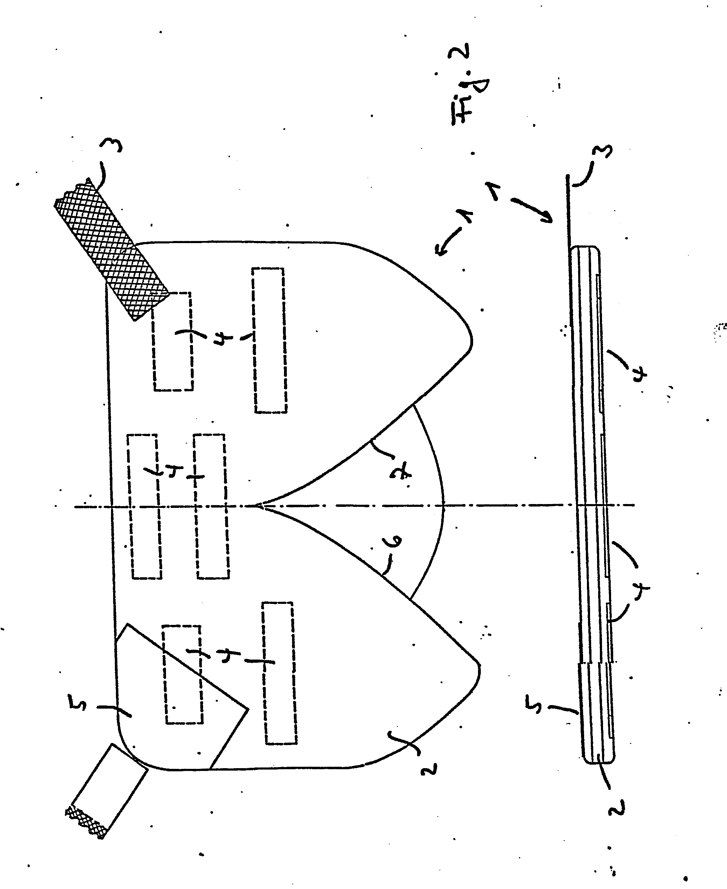

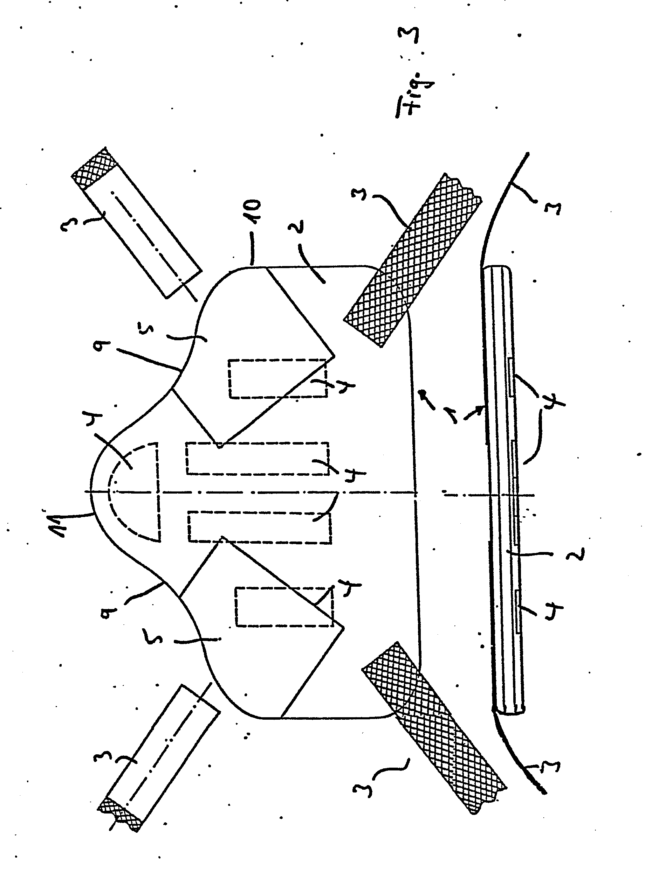

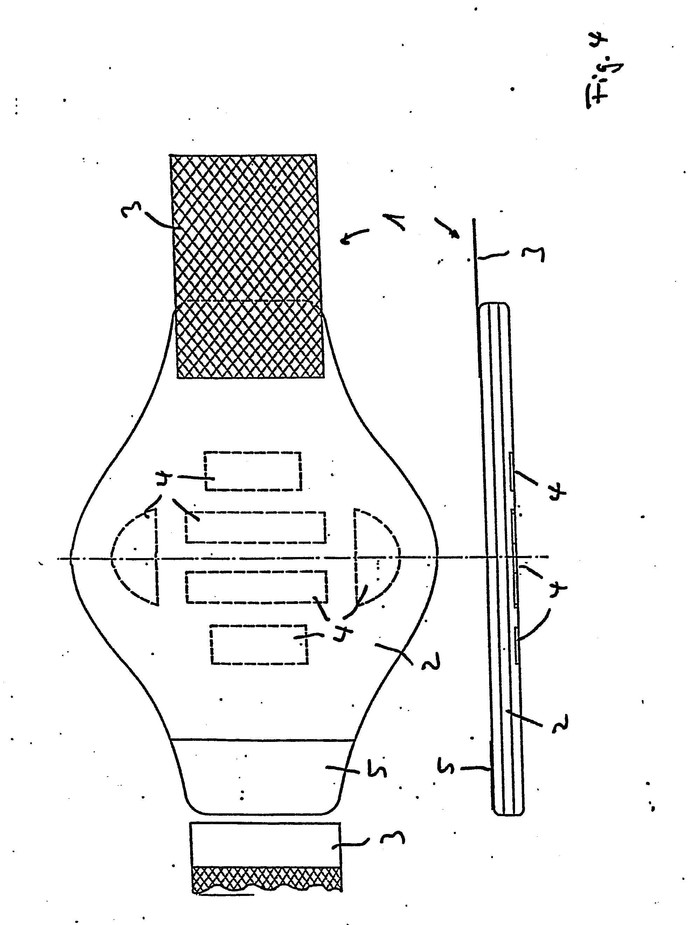

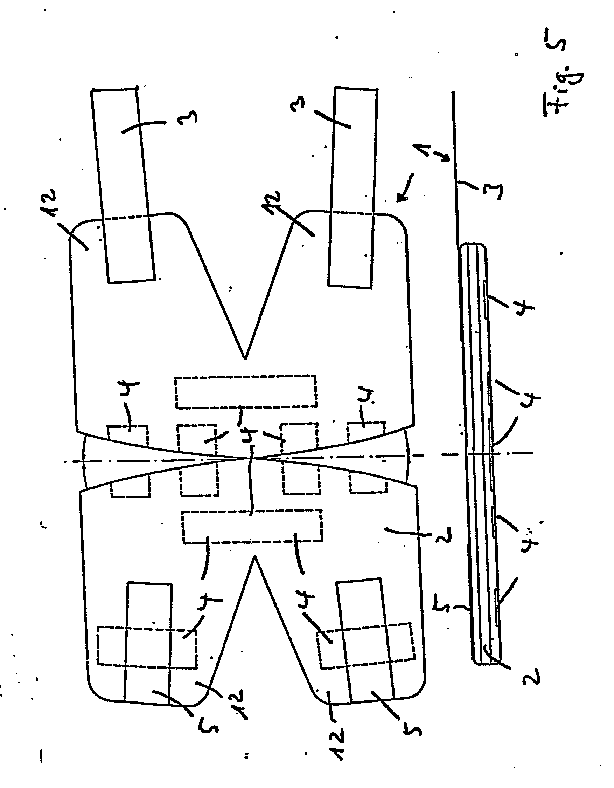

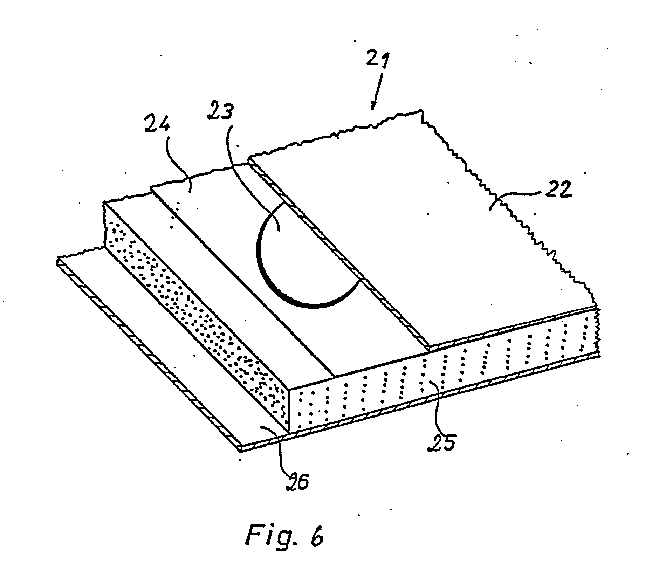

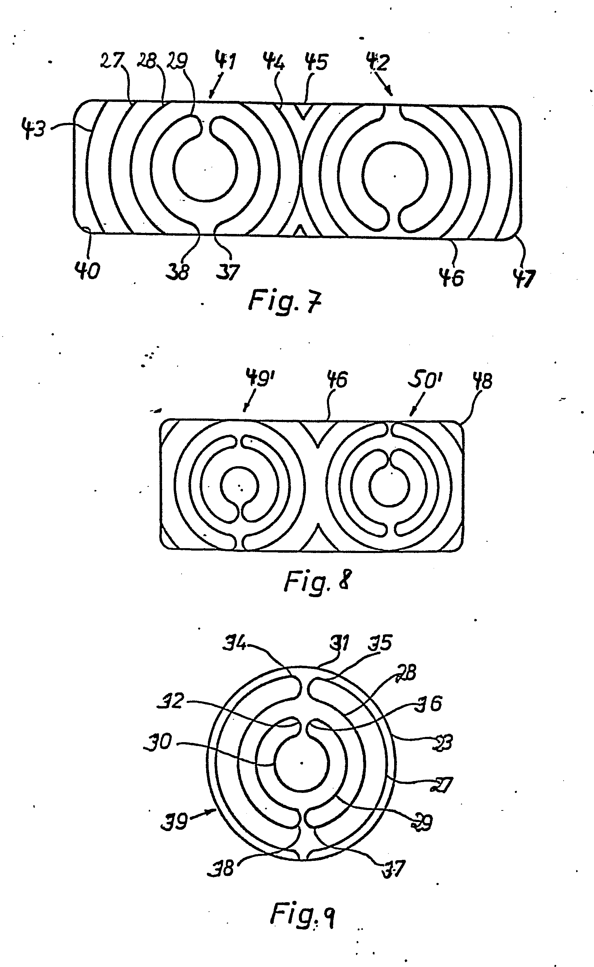

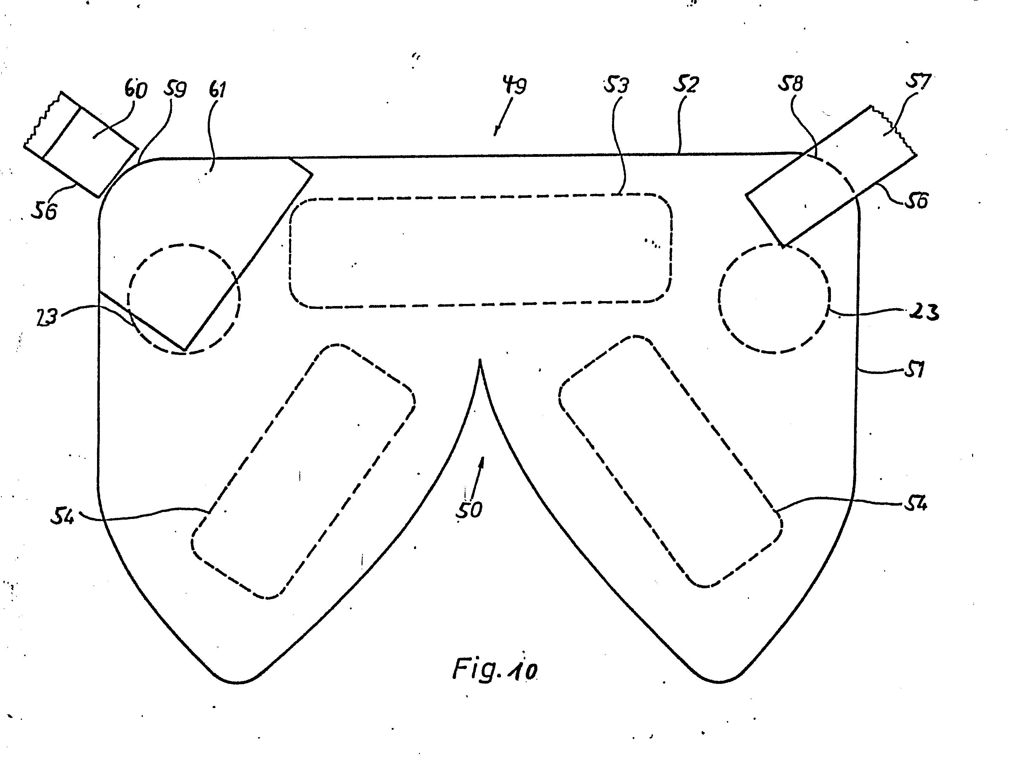

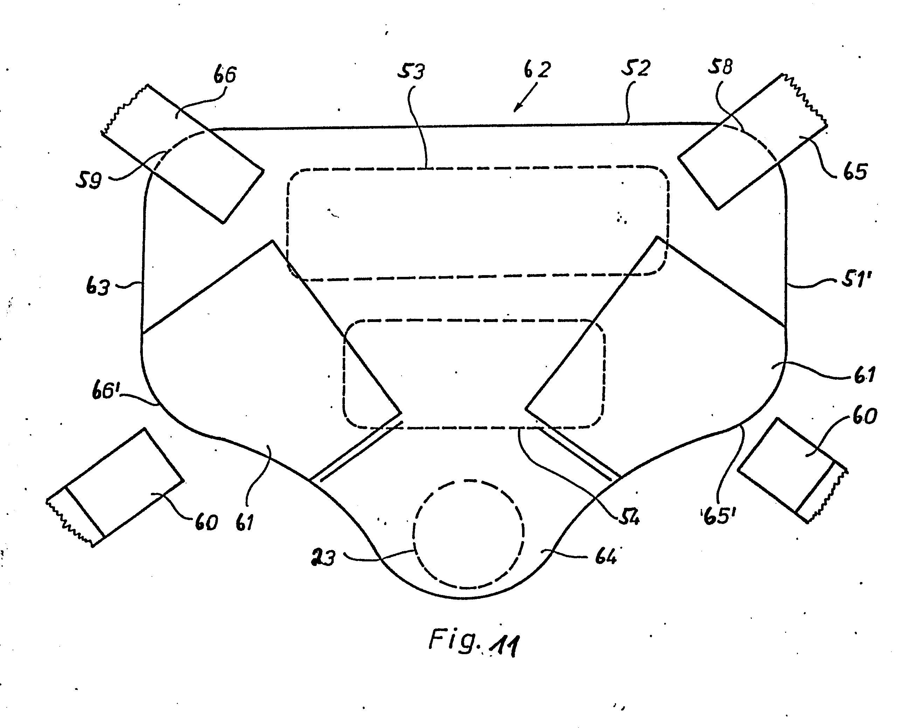

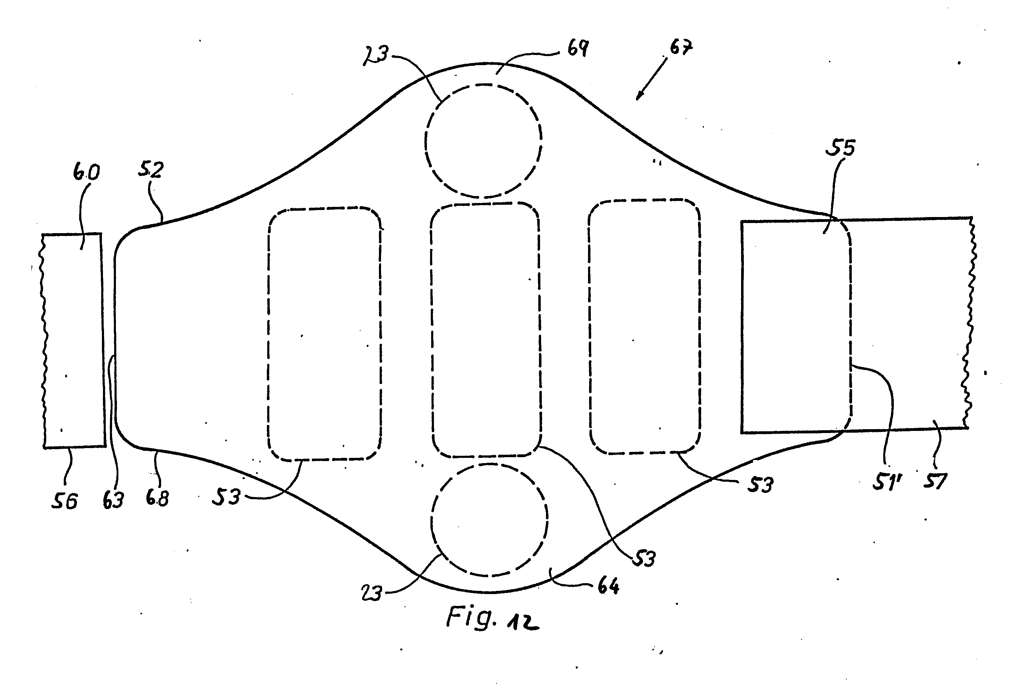

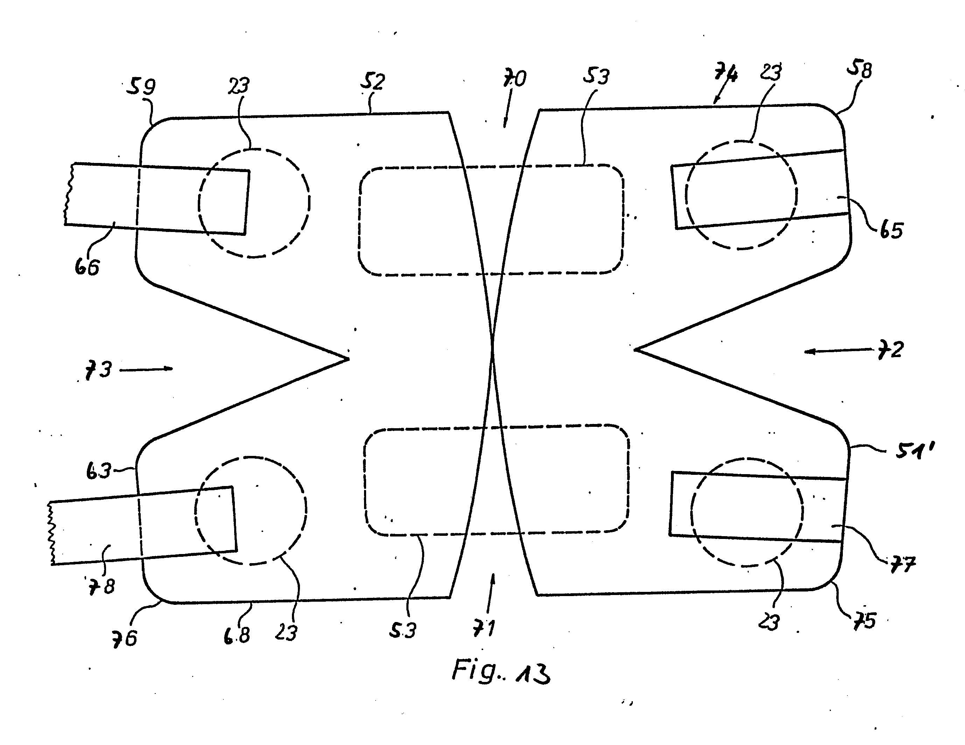

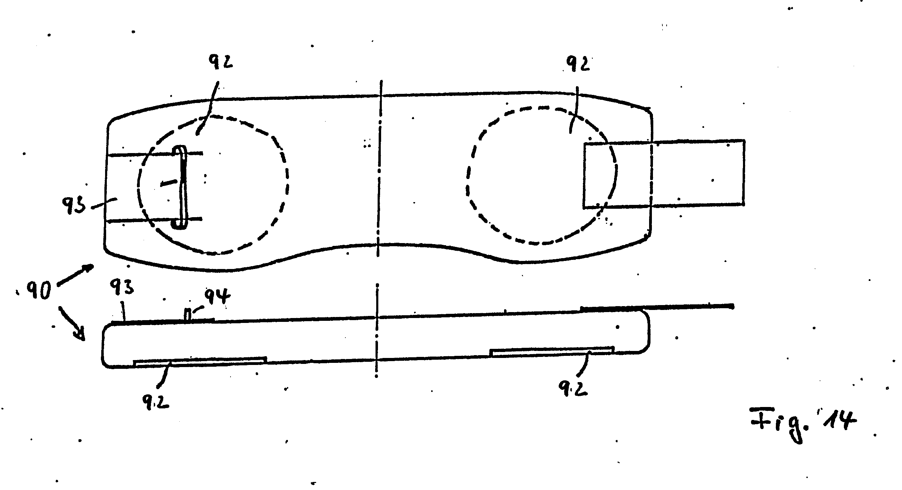

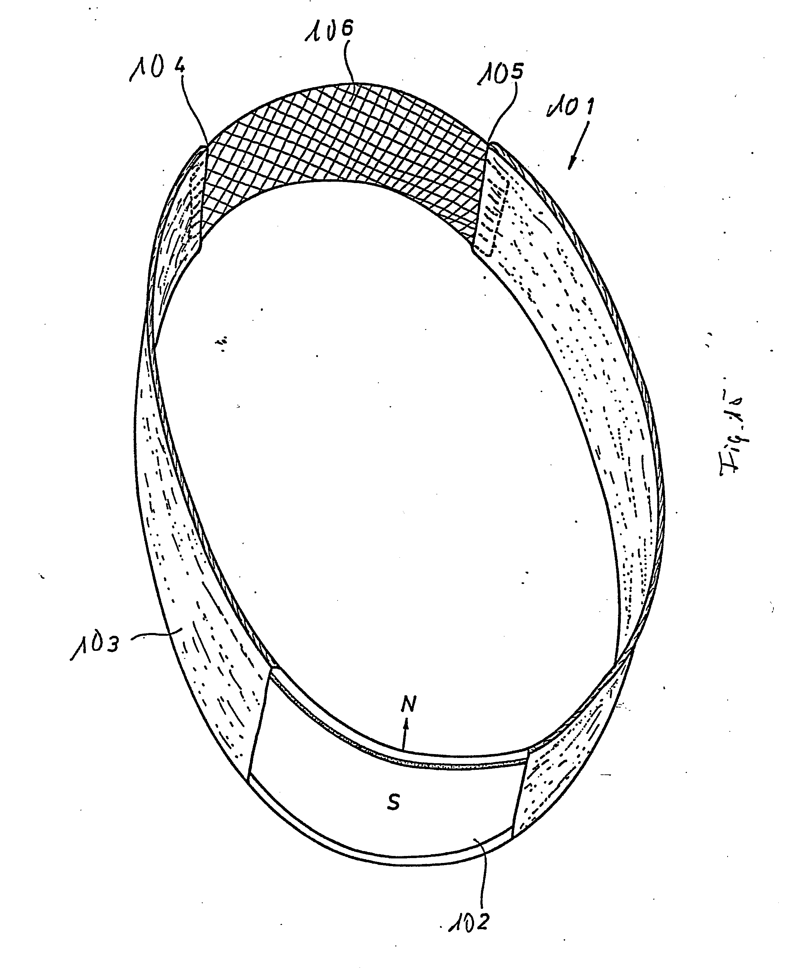

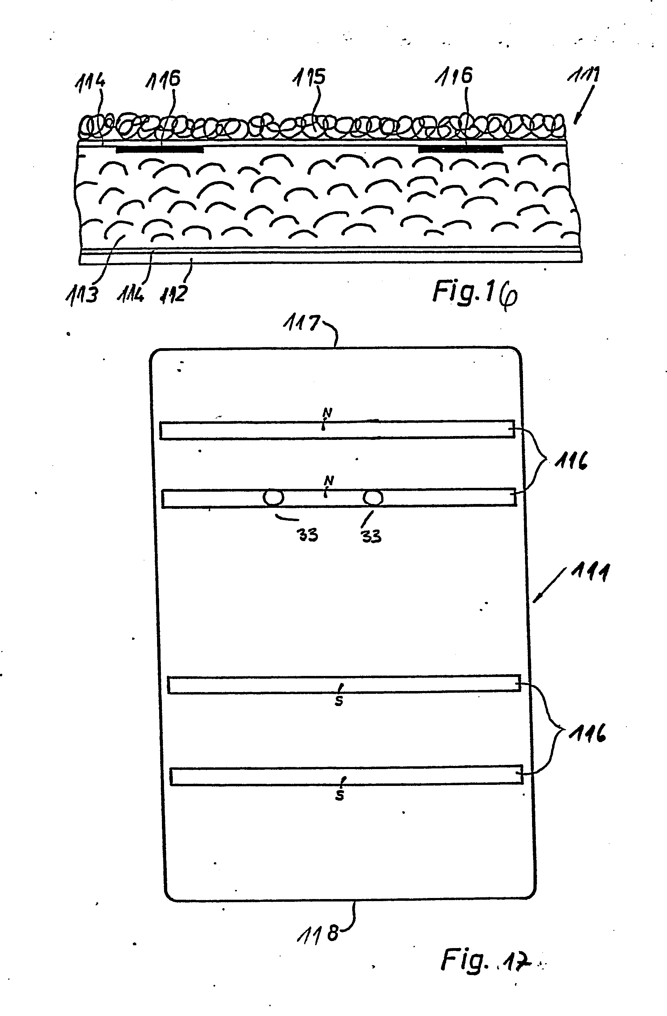

In den Fig. 1 bis 5 ist die Bandage jeweils mit dem Bezugszeichen (1) versehen. Alle Bandagen bestehen aus einem Bandagenkörper (2) und daran einseitig befestigten Bändern (3). Sämtliche Bandagenkörper sind schichtförmig ausgebildet. Die zur behandelnde Körperstelle zeigende Fläche des Bandagenkörpers besteht aus einem Baumwollgewebe. Die von der Körperstelle wegweisende Schicht des Bandagenkörpers (2) besteht aus einem Brushednylongewebe. Zwischen diesen beiden Lagen ist eine Schicht aus einem faserförmigen Vlies angeordnet. Das faserförmige Vlies, im folgenden auch Thermovlies genannt, besteht aus einem offenzelligen Schaumstoff. In das Thermovlies und zwar an der Übergangsstelle von Thermovlies zu Baumwollgewebe sind im Bandagenkörper (2) mehrere folienförmige flexible Magnetelemente (4) angeordnet.

Die Fig. 1 zeigt eine Bandage anzulegen bei Epicondylitis, auch Tennisarm genannt. Der Bandagenkörper (2) ist in etwa rechteckförmig ausgebildet und so bemessen, daß er den zu behandelnden Unterarm nicht ganz umschließt. An der in der Zeichnung rechten Seite ist das Befestigungsband angenäht und wird mit Hilfe eines Klettverschlusses (5) am linken Rand des Bandagenkörpers (2) befestigt. In der Zeichnung ist das Band als rechteckiger Streifen dargestellt. Es kann jedoch auch so ausgestaltet sein, daß es konkave Umrisse aufweist, derart, daß das Band (3) bandagenkörperseitig in etwa so breit ist wie die Schmalseite des Bandagen körpers selbst, wonach die Breite kontinuierlich abnimmt und zum Ende des Bandes wieder kontinuierlich zunimmt. Im linken Drittel und im rechten Drittel des Bandagenkörpers (3) sind die Magnetelemente (4) angeordnet.

Die Fig. 1 a stellt eine alternative Ausführungsform der Epicondylitis-Bandage dar. Zur Verstärkung des Wärmeffekts sind an jeder Seite der Bandage zwei Magnete (4) angeordnet. Damit die Bandage gleichzeitig auch als Druckverband wirken kann, wird das Band (3) neben dem Klettverschluß (5) noch durch eine Schnalle (5') am Bandagenkörper (2) befestigt. Darüberhinaus sind die beiden Enden des Bandagenkörpers (2) verbreitert, so daß sich im Mittelbereich des Körpers (2) einseitig eine Einbuchtung (20) ergibt, die der inneren Armbeuge angepaßt ist.

In der Fig. 2 ist eine Bandage für das Schultergelenk dargestellt. Gestrichelt sind die Magnetelemente (4) dargestellt. In der rechten oberen Ecke ist das Band (3) befestigt, welches abgebrochen dargestellt ist. Das freie Ende des Bandes (3) ist dagegen an der linken oberen Ecke der Bandage dargestellt. Auf dieser Ecke der Bandage wird das freie Ende der Bandage mit Hilfe eines Klettverschlusses (5) befestigt. Der Bandagenkörper (2) ist aufgeschnitten dargestellt. Im zusammengenähten Zustand, d. h., wenn die zwei mittig zusammenlaufenden Kanten (6 und 7) zuammengenäht sind, bildet der Bandagenkörper (2) eine Tasche, die an die Schulterpartie angepaßt ist.

In der Fig. 3 ist eine Bandage (1) dargestellt, die zur Anwendung im Schulternackenbereich vorgesehen ist. An den beiden unteren Kanten des Bandagenkörpers (2) greifen schräg die Bänder (3) an, die kreuzförmig um den Brustkorb herumgeschlungen werden und mit ihren freien Enden an den oberen Schrägseite (8 und 9) des Bandagenkörpers mit Hilfe der Klettverschlüsse (5) befestigt werden. Der Bandagenkörper (2) deckt mit einem größeren Bereich (10) die Körperpartie zwischen den beiden Schultern und mit einem kleineren in etwa halbkreisförmigen Bereich (11) den Nackenbereich ab. Die Magnetelemente (4) sind wiederum gestrichelt dargestellt.

In der Fig. 4 ist eine Bandage für den Rücken- bzw. Lendenbereich dargestellt. Das Band (3) ist an beiden Rändern des Bandagenkörpers (2) befestigt und wird um die Bauch-Brustpartie geschlungen und mit Hilfe des Klettverschlusses vorne auf der Bandseite befestigt. Auch hier sind die Magnetelemente mit dem Bezugszeichen (4) versehen und gestrichelt dargestellt.

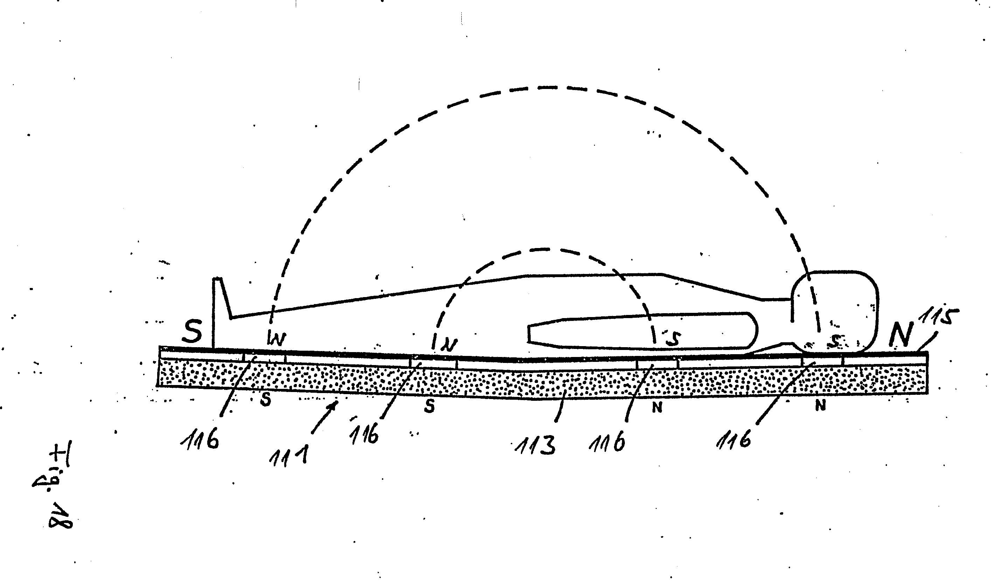

Die Fig. 5 stellt eine Bandage für den Ellenbogen bzw. für die Knie-oder die Knöchelgelenkspartie dar. Die in etwa X-förmig zusammenlaufenden Kanten in der Mitte der Darstellung werden zusammengenäht, so daß sich ein etwa muldenförmiges Gebilde ergibt, welches sich den Beugen im Ellenbogen-, im Knie-bzw. im Knöchelgelenksbereich anpaßt. Der Bandagenkörper (2) läuft am rechten bzw. linken Rand in vier Lappen (12) aus. Jeweils zwei Lappen an einer Seite des Bandagenkörpers (2) sind durch einen V-förmigen Ausschnitt voneinander getrennt. An dem rechtsseitigen Lappen ist jeweils ein Band (3) befestigt, deren freie Enden mit Hilfe des Klettverschlusses (5) an den entsprechenden linksseitigen Lappen (12) befestigt werden. Auch hier sind wie bei allen anderen Ausführungsbeispielen die Magnetelemente (4) gestrichelt dargestellt. Im Gegensatz zu den Ausführungsbeispielen gemäß Fig. 2 bis 4 sind die Bänder (3) wie beim Ausführungsbeispiel gemäß Fig. 1 unelastisch ausgebildet.

Sämtliche dergestellten Bandagen werden so angelegt, daß der Bandagenkörper (2) mit seiner Thermovlieseinlage und mit den darin angeordneten Magnetelementen (4) auf der erkrankten Körperstelle zu Liegen kommt, so daß die Bereiche der größten Wärmeentwicklung gerade im Bereich der erkrankten Körperstelle positioniert sind.

Aus Fig. 6 ist der Aufbau der Bandage (21) ersichtlich. Dieser ist schichtförmig. Der körpernahe Bereich der Bandage wird von einem auf der Haut aufliegenden, Schweiß aufnehmenden Baumwollgewebe (22) gebildet. Hieran schließt sich eine nicht kontinuierliche, sondern aus einzelnen Abschnitten bestehende Schicht an, die von Magnetfolien gebildet wird, die in der Darstellung der Fig. 6 kreisscheibenförmigen Umriß aufweisen und mit (23) bezeichnet sind. Diese Magnetfolien liegen auf der Innenseite einer Nickelfolie (24), welche im Ausführungsbeispiel aus einer Nickelkobaltlegierung besteht, in der das Nickel den überwiegenden Anteil von ca. 99,6 % ausmacht; eine solche Nickelfolie hat gut mechanische Eigenschaften und entwickelt einen hervorragenden Widerstand gegen korrosive Sphären. An die Metallfolienschicht (24) schließt sich nach außen eine wärmespeichernde Vliesschicht (25) an, deren offene Poren in der Darstellung der Fig. 6 angedeutet sind. Die Vliesschicht ist außen mit einem Kunststoffgewebe (26) abgedeckt, welches gemäß dem Ausführungsbeispiel aus Brushednylon besteht.

Die Magnetfolie (23) ist in Fig. 9 mit ihrer Magnetisierung wiedergegeben. Danach ist die Masse der Magnete in konzentrischen Ringen (30) angeordnet. Die Ringe verlaufen im wesentlichen parallel zur Umrißlinie (31) der Scheibe (23). Zum geringeren Teil sind die Magnete längs bogenförmiger, gemäß dem Ausführungsbeispiel über einen halben Kreisbogen reichender Bahnen (32-38) angeordnet. Diese Bahnen verbinden jeweils benachbarte Ringe im geringen Abstand voneinander, wobei mehrere Verbindungen einander benachbart sind. Die Anordnung ist dabei so getroffen, daß der innere Ring (30) mit Hilfe der Bahnen (32 und 36) mit dem nach außen folgenden Ring (29) verbunden ist, der seinerseits mit den Bahnen (37 und 38) eine Verbindung mit dem folgenden Ring (28) aufweist, der wiederum mit den Bahnen (34 und 35) eine Verbindung mit dem außenliegenden Ring (27) aufweist. Es ergibt sich dadurch eine magnetische Reihenschaltung der Magnete und die Anordnung der magnetisierbaren Eisenteile im wesentlichen längs ringförmiger Bahnen, welche das magnetische Feld konzentriert. Diese Zirkularmagnetisierung (39) ist hauptsächlich für die verbesserte therapeutische und prophylaktische Wirkung verantwortlich, welche eingangs beschrieben worden ist, was auf der durch die beschriebene Anordnung und der dadurch u. a. eintretenden Verwirbelung der magnetisch beeinflußbaren körpereigenen Elemente bzw. Substanzen beruht.

Die Fig. 7 und 8 zeigen, wie man die Wirkung der Zirkularmagnetisierungen auf ausgedehntere Körperregionen konzentrieren kann.

Gemäß dem Ausführungsbeispiel der Fig. 7 weist die Magnetfolie (40) zwei Zirkularmagnetisierungen (41, 42) nach Art der Zirkularmagnetisierung (39) auf, die im Zusammenhang mit der Fig. 9 vorstehend beschrieben worden ist. Unterschiedlich ist, daß die gekrümmten Verbindungsbahnen (37, 38) der Ringe (28, 29) zum Teil und die Verbindungsbahnen (34, 35) ganz durch den Folienabschnitt unterbrochen sind, während außenliegende Ringsegmente (43-45) über dem Ausführungsbeispiel der Fig. 9 zusätzlich vorhanden sind. Im übrigen sind die Zirkularmagnetisierungen (41 und 42) identisch.

Das ist auch beim Ausführungsbeispiel der Fig. 8 der Fall, bei der die Ringabstände geringer sind, so daß trotz der gegenüber der Fig. 7 geringeren Fläche bei rechteckigem Umriß (46) des Folienabschnittes mit abgerundeten Ecken, wie bei (47) in Fig. 7 und (48) in Fig. 8 dargestellt, eine größere Anzahl von hintereinander geschalteten Magneten vorhanden ist. Die Anzahl, Anordnung und Ausbildung der Ringe der Zirkularmagnetisierungen (49', 50') im Ausführungsbeispiel der Fig. 8 entsprechen im übrigen der Zirkularmagnetisierung (39) im Ausführungsbeispiel der Fig. 9.

In der Schultergelenkbandage (49) nach Fig. 10 sind in dem allgemein rechteckigen, jedoch mit einem tiefen Einschnitt (50) versehenen Zuschnitt (51) der Bandage längs der längeren Bandagenseite (52) eine Magnetfolie (53) gemäß dem Ausführungsbeispiel der Fig. 2 und zwei kreisscheibenförmige Magnetfolien (23) nach Art des Ausführungsbeispiels der Fig. 9 untergebracht. Längs des Eischnittes (50) auf der gegenüberliegenden Rechteckseite befindet sich je eine Magnetfolie (54) gemäß dem Ausführungsbeispiel der Fig. 8. Die freien Enden (55, 56) eines Bandagenbandes (57) sind in den oberen Ecken (58, 59) des Bandagengrundrisses an der längeren Rechteckseite (52) angeordnet, wobei das Ende (56) mit einem Klettverschluß (60, 61) versehen ist. Die Einschnittkanten werden bei der Konfektion der Bandage miteinander vernäht.

Bei der für den Schulter-Nackenbereich vorgesehenen Bandage (62) im Ausführungsbeispiel der Fig.11 wird ebenfalls ein allgemein reckteckiger Grundriß mit einer oben liegenden, längeren Rechteckseite (52) verwendet. Die kürzeren Rechteckseiten (51', 63) gehen jedoch in eine nach außen gerichtete angeschnittene Lasche (64) über. Zwei Bandagenbänder (65, 66) sind einerseits in den oberen Ecken (58, 59) fest und andererseits mit Klettverschlüssen (60, 61) versehen, die in den unteren Ecken (65', 66') des reckteckförmigen Zuschnittes angeordnet sind.

Die Lasche (64) dient zur Unterbringung einer kreisscheibenförmigen Magnetfolie (23), über der eine rechteckige Magnetfolie (54) gemäß dem Ausführungsbeispiel der Fig. 8 und eine ebenfalls rechteckförmige Magnetfolie (53) gemäß dem Ausführungsbeispiel der Fig. 7 angeordnet sind. Die längeren Rechteckseiten verlaufen wiederum parallel zur längeren Rechteckseite (52) des Bandagenanschnittes.

Im Ausführungsbeispiel der Fig. 12 ist die für den Rücken-und Lendenbereich vorgesehene Bandage (67) an ihren längeren Rechteckseiten (52, 68) mit je eine Lasche (64, 69) versehen. In den Laschen liegt jeweils eine kreisscheibenförmige Magnetfolie (23) gemäß dem Ausführungsbeispiel der Fig. 9. Parallel zu den kürzeren Rechteckseiten (63, 61') liegen im gleichen Abstand voneinander rechteckförmige Magnetfolienabschnitte (53) gemäß der Ausführungsform nach Fig. 7. Insgesamt sind drei Folienabschnitte (53) vorhanden. Ein Bandagenband (57) ist an der kürzeren Rechteckseite (51'), wie bei (55) in Fig. 10 dargestellt, befestigt und an seinem freien Ende (56) mit dem Klettverschluß (60) versehen.

Im Ausführungsbeispiel der Fig. 13 sind sowohl die längeren Seiten (52, 68), wie auch die kürzeren Seiten (51', 63) des rechteckförmigen Bandagenzuschnittes mit Einschnitten (70, 71 bzw. 72, 73) versehen, um die fertige Bandage (74) um ein Ellenbogen-, Knie-oder Knöchelgelenk herumlegen zu können, wenn die Kanten der Einschnitte (70-73) vernäht sind. In den vier Ecken (58, 59 und 75, 76) des recheckigen Grundrisses liegt jeweils ein kreisscheibenförmiger Abschnitt (23) einer Magnetfolie gemäß dem Ausführungsbeispiel der Fig. 9. Parallel zu den längeren Recteckseiten (52 und 68) erstrekken sich Magnetfolien (53) gemäß dem Ausführungsbeispiel der Fig. 7.

Im Ausführungsbeispiel der Fig. 13 werden wie im Ausführungsbeispiel der Fig. 11 zwei Bandagenbänder mit ihren Enden (65, 66 bzw. 77, 78) zur Befestigung der Bandage auf der betreffenden Körperregion verwendet.

Wie sich aus den Ausführungsbeispielen der Fig. 11 bis 13 ergibt, werden durchweg zirkular magnetisierte Folienabschnitte benutzt, deren Ausbildung, Größe und Anordnung im einzelnen von dem Zuschnitt der Bandage abhängig ist, der seinerseits auf der Form und Ausdehnung der Körperregion beruht, welche bandagiert werden soll.

Die Fig. 14 zeigt eine Bandage- anzulegen bei Epicondylitis, auch Tennisarm genannt. Der Bandagenkörper (90) ist in etwa rechteckförmig ausgebildet und so bemessen, daß er den zu behandelnden Unterarm nicht ganz umschließt. An der in der Zeichnung rechten Seite ist das Befestigungsband angenäht und wird mit Hilfe eines Klettverschlusses (93) am linken Rand des Bandagenkörpers (90) befestigt. In der Zeichnung ist das Band als rechteckiger Streifen dargestellt. Es kann jedoch auch so ausgestaltet sein, daß es konkave Umrisse aufweist, derart, daß das Band bandagenkörperseitig in etwa so breit ist, wie die Schmalseite des Bandagenkörpers selbst, wonach die Breite kontinuierlich abnimmt und zum linken Ende des Bandes wieder kontinuierlich zunimmt. Im linken Drittel und im rechten Drittel des Bandagenkröpers (90) sind die Magnetelemente (92) angeordnet. Damit die Bandage gleichzeitig auch als Druckverband wirken kann, wird das Band neben dem Klettverschluß (93) noch durch eine Schnalle (94) am Bandagenkörper (90) befestigt. Darüberhinaus sind die beiden Enden des Bandagenkörpers (90) verbreitert, so daß sich im Mittelbereich des Körpers (90) einseitig eine konkave Ausbuchtung (91) ergibt, die der inneren Armbeuge angepaßt ist. Die kreisförmigen Magnetfolien weisen eine Dicke von etwa 1,5 mm auf und eine Magnetfeldstärke, die im Zentrum etwa 610 Gaus und in der Peripherie etwa 590 Gaus beträgt.

In der Fig. 15 ist ein Stirnband insgesamt mit dem Bezugszeichen (101) versehen. Das Stirnband (101) besteht aus einem Streifen einer axialmagnetisierten Folie (102) und einem diese Folie (102) umgebenden Stoffüberzug (103). Der Stoffüberzug (103) umschließt die gesamte Folie (102a). Lediglich aus Übersichtlichkeitsgründen ist in der Zeichnung im Stirnbereich die Magnetfolie (102) freigelegt. An den beiden Enden (104 und 105) der mit dem Stoffüberzug (103) umhüllten Magnetfolie (102) ist ein elastisches Band (106) mit seinen beiden Enden befestigt. Die axialmagnetisierte Folie (107) ist dabei so im Stoffüberzug (103) angeordnet, daß die Obefläche, die den magnetischen Südpol trägt, nach außen zeigt, währen der magnetische Nordpol zum Zentrum des Stirnbandes (109) weist. Infolge des elastischen Bandes (106) ist das Stirnband praktisch jeder Kopfgröße anpaßbar, wobei die Länge der Magnetfolie (102) so bemessen ist, daß sie in jedem fall die Stirn-und Schläfenpartie bis zum Ohrbereich umschließt.

Der Stoffüberzug (103) ist ein Frotteegewebe aus natürlichen hochwertigen Ringgarnen.

In den Figuren ist die Decke allgemein mit dem Bezugszeichen (111) versehen. Wie insbesondere die Fig. 16 zeigt, ist die Decke schichtweise aufgebaut. Als untere Schicht dient ein Baumwollgewebe (112), welches durch die Rauhigkeit der Gewebeart eine Anti-Rutschwirkung aufweist. Außerdem gewährleistet das Baumwollgewebe durch seine Atmungsaktivität, daß die Decke nich im Laufe einere längeren Benutzungsdauer durch kondensierende Körperfeuchtigkeit unansehnlich bzw. zur Brutstätte von Krankheitskeimen wird. Über der Baumwollgewebsschicht (112) ist eine etwa 2 cm dicke Schicht aus Polyester-Vlies (113) angeordnet. Dieses Polyester-Vlies (113) wird durch einen offenzelligen, bei der Herstelung aerodynamisch geblasenen Schaumstoff gebildet, der die Fähigkeit hat, sich auch nach längerer Belastung wieder voll aufzurichten. Somit bleiben die vielen Zwischenräume erhalten, somit auch die Isolationsfähigkeit, d. h. die Temperaturausgleichfähigkeit. Zur Erhöhung der Strapazierfähigkeit und zur Erhöhung der Isolationsfähigkeit ist das Polyester-Vlies an beiden Oberflächen mit einer Harzschicht (114) besprüht. Die Harzschicht (114) ist dabei extrem dünn und daher in der Zeichnung übertrieben dargestellt. Im oberen Bereich der Polyester-Vliesschicht (113), und zwar angrenzend an die oberste Schicht der Decke, einer Lage (115) aus Schafwolle, sind in Querrichtung der Decke folienförmige flexible Magnetelemente (116) angeordnet. Diese Folien sind so magnetisiert, daß eine Oberfläche den magnetischen Nordpol und die ihr gegenüberliegende Oberfläche den magnetischen Südpol bildet. Kopfendseitig sind die Magnetelemente (116) so in der Decke angeordnet, daß die den magnetischen Südpol bildenden Oberflächen der Magnetelemente zum Körper des auf der Decke Ruhenden weisen. Die fußendseitigen Magnetelemente (116) weisen mit ihrem magnetischen Nordpol zum auf der Decke Ruhenden. Somit ergibt sich ein Magnetfeld, daß das natürliche Erdmagnetfeld simuliert. Der Ruhende liegt unabhängig von der Orientierung der Decke im Raum immer in einer simulierten geografischen Nord-Süd-Richtung. Somit kann auch in Zimmern, in denen die Stellung des Bettes in Nord-Süd-Richtung nicht möglich ist, diese für den Schlaf besonders geeignete Stellung eingehalten werden, da das natürliche Magnetfeld im Vergleich zu dem künstlichen Magnetfeld vernachlässigt werden kann.

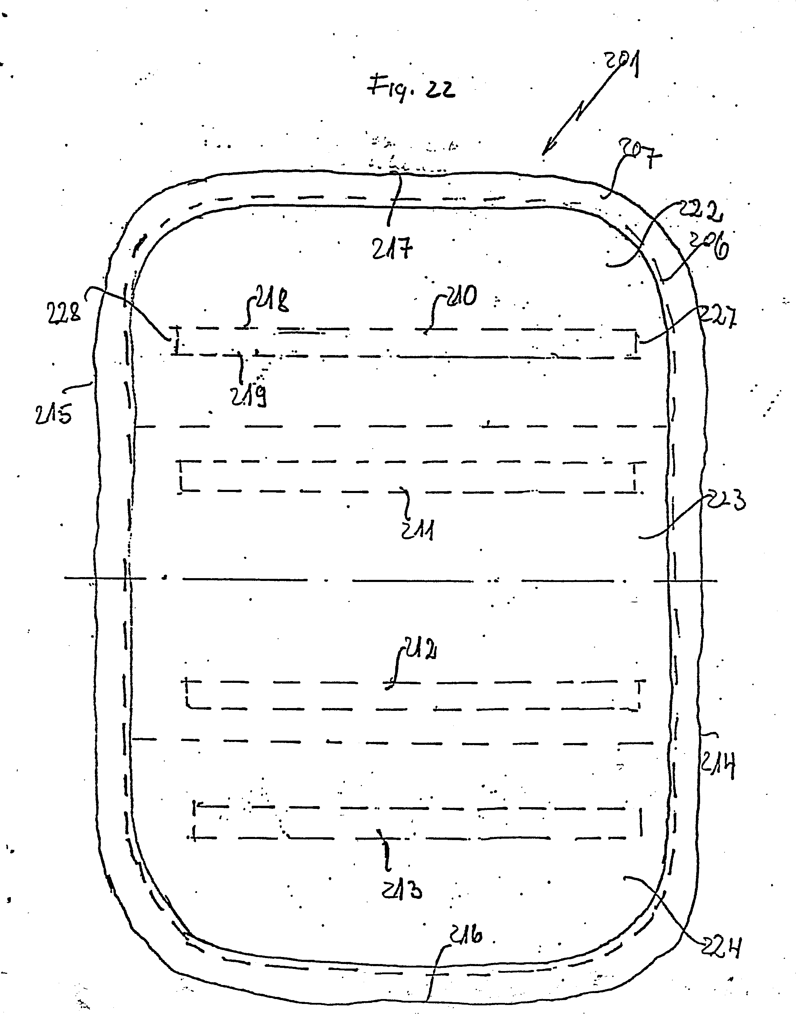

In der Fig. 19 ist in abgebrochener Darstellung der Bereich der Zirkularmagnetisierung auf den Magnetelementen (116) dargestellt. Das Prinzip der Zirkularmagnetisierung geht aus der Fig. 21 hervor. Danach ist die Masse der Magnete in konzentrischen Ringen (120) angeordnet. Die Ringe verlaufen im wesentlichen parallel zur Umrißlinie (121) der Scheibe. Zum geringeren Teil sind die Magnete längs bogenförmiger, gemäß dem Ausführungsbeispiel über einen halben Kreisbogen reichender Bahnen (122-128) angeordnet. Diese Bahnen verbinden jeweils benachbarte Ringe im geringen Abstand voneinander, wobei mehrere Verbindungen einander benachbart sind. Die .Anordnung ist dabei so getroffen, daß der innere Ring (120) mit Hilfe der Bahnen (123 und 126) mit dem nach außen folgenden Ring verbunden ist, der seinerseits mit den Bahnen (127 und 128) eine Verbindung mit dem folgenden Ring aufweist, der wiederum mit den Bahnen (124 und 125) eine Verbindung mit dem außenliegenden Ring aufweist.

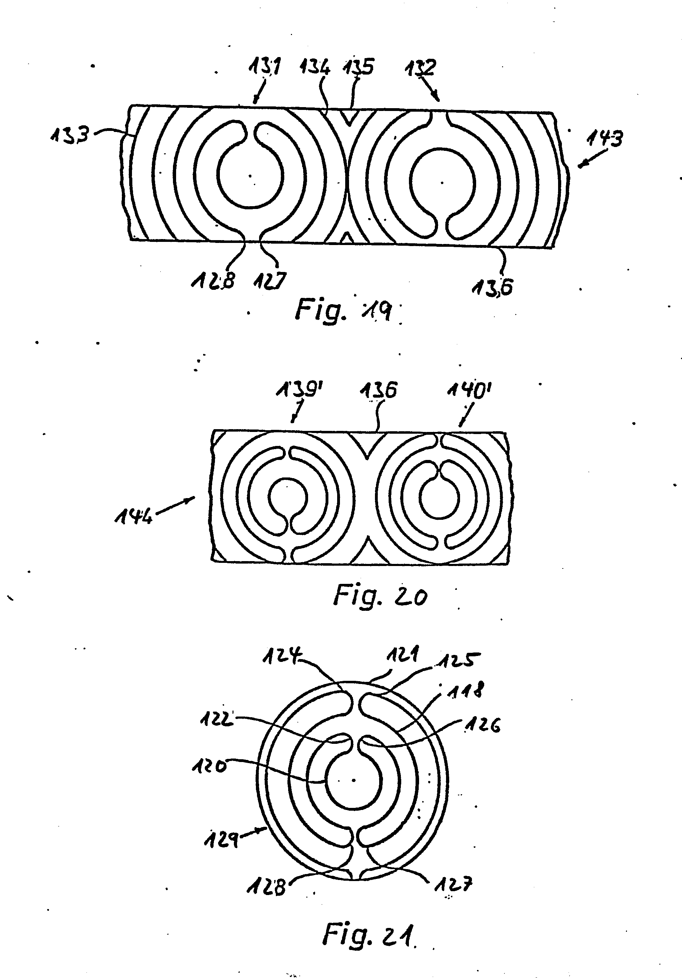

Es ergibt sich dadurch eine magentische Reihenschaltung der Magnete und die Anordnung der magentisierbaren Eisenteile im wesentlichen längs ringförmiger Bahnen, welche das magnetische Feld konzentriert. Diese Zirkularmagnetisierung (129) ist hauptsächlich für die verbesserte therapeutische und prophylaktische Wirkung verantwortlich, welche eingangs beschrieben worden ist, was auf der durch die beschriebene Anordnung und der dadurch u. a. eintretenden Verwirbelung der magnetisch beeinflußbaren körpereigenen Elemente bzw. Substanzen beruht. Gemäß dem Ausführungsbeispiel der Fig. 19 weist die Magnetfolie (116) zwei Zirkularmagnetisierungen (131, 132) nach Art der Zirkularmagnetisierung (129) auf, die im Zusammenhang mit der Fig. 20 vorstehend beschrieben worden ist. Unterschiedlich ist, daß die gekrümmten Verbindungsbahnen (127, 128) der Ringe z. T. und die Verbindungsbahnen (124, 125) der dazwischenliegenden Ringe ganz durch den Folienanschnitt unterbrochen sind, während außenliegende Ringsegmente (133, 135) über dem Ausführungsbeispiel der Fig. 19 zusätzlich vorhanden sind. Im übrigen sind die Zirkularmagnetisierungen (131 und 132) identisch. Das ist auch beim Ausführungsbeispiel der Fig. 21 der Fall, bei der die Ringabstände geringer sind, so daß trotz der gegenüber der Fig. 20 geringeren Fläche bei rechteckigem Umriß (130) des Folienabschnittes eine größere Anzahl von hintereinandergeschalteten Magneten als beim Beispiel der Fig. 19 vorhanden ist. Die Anzahl und Ausbildung der Ringe der Zirkularmagnetisierungen (139, 140) im Ausführungsbeispiel der Fig. 21 entsprechen im übrigen der Zirkularmagnetisierung (129) im Ausführungsbeispiel der Fig. 21.

In der Fig. 22 ist ein allgemein mit (201) bezeichnetes Flachkissen wiedergegeben, welches einen allgemein rechteckigen Umriß aufweist, der sich einer zu bandagierenden Körperpartie, z. B. der Gruppe eines Pferdes anpaßt. Dieser Umriß paßt sich außerdem der Bandage an, die aus einer Decke be steht, welche mehrere Taschen zur Unterbringung je eines Flachkissens der aus Fig. 22 ersichtlichen Art aufweist.

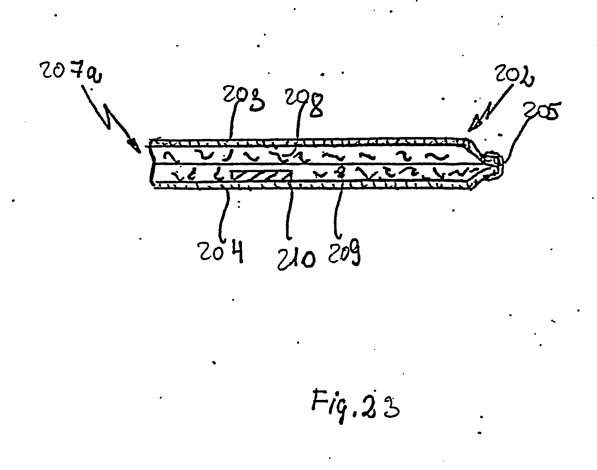

Das Flachkissen (201) hat einen Kissenbezug (202), welcher aus zwei kongruenten Baumwollstoffabschnitten (203, 204) besteht. Diese sind deckungsgleich aufeinanderliegend angeordnet und an ihren Rändern, wie bei (205) in Fig. 23 dargestellt, miteinander verbunden. Die Verbindung besteht aus einer umlaufenden Steppnaht (206) und eine äußeren Einfassung (207), welche mit der Steppnaht gehalten wird. In dem Kissenbezug befindet sich eine Kissenfüllung (207 a). Sie besteht aus einem Paar von kongruenten Vliesausschnitten (208, 209), die zusammen eine Doppellage des Vlieses ergeben, welche den Umriß der Stoffausschnitte (203, 204) aufweist. An den Rändern ist die Doppellage mit der Steppnaht (207) an die beschriebenen Teile des Kissens angeschlossen.

Ferner besteht die Kissenfüllung (207) aus mehreren Magnetstreifen (210-213), die quer zur Wickelrichtung des Umschlages ausgerichtet sind, welche gemäß dem dargestellten Ausführungsbeispiel längs der Längskanten (214, 215) des Kissen verläuft, so daß die Magnetstreifen parallel zu den kurzen Kanten (216, 217) des Kissenumrisses angeordnet sind.

Die Magnetstreifen (210-213) verlaufen daher im wesentlichen über die gesamte Breite des Kissens (201). Ihr Grundriß ist rechteckig, wobei die Längskanten (218, 219) parallel zu Steppnähten (220, 221) verlaufen, die das Kissen in drei Abschnitte (222-224) unterteilen. Jedem der äußeren Kissenabschnitte (222, 224) ist ein Folienstreifen (210, 213) zugeordnet, während der mittlere Kissenabschnitt (223) die beiden Folienstreifen (211 und 212) enthält. Die Anordnung ist dabei so getroffen, daß die Längsmitte der Folienstreifen (210-213) etwa mit der Mitte der betreffenden Kissenabschnitte (222, 224) zusammenfällt, wenn die Folienstreifen (211, 212) des mittleren Kissenabschnittes (223) symmetrisch zu dessen querverlaufender Mittellinie angeordnet sind. Dabei sind die kurzen Folienstreifenkanten (227, 228) parallel zu den benachbarten Kissenrändern (214, 215) und unmittelbar neben der Randsteppnaht (206) angeordnet.

Außen trägt das Kissen auf seinem Stoffausschnitt (204) eine Markierung, die sicherstellt, daß die Folienstreifen, welche mit einer Seite der Innenseite des Stoffausschnittes (204) anliegen, unmittelbar über der zu behandelnden Körperpartie angeordnet werden.

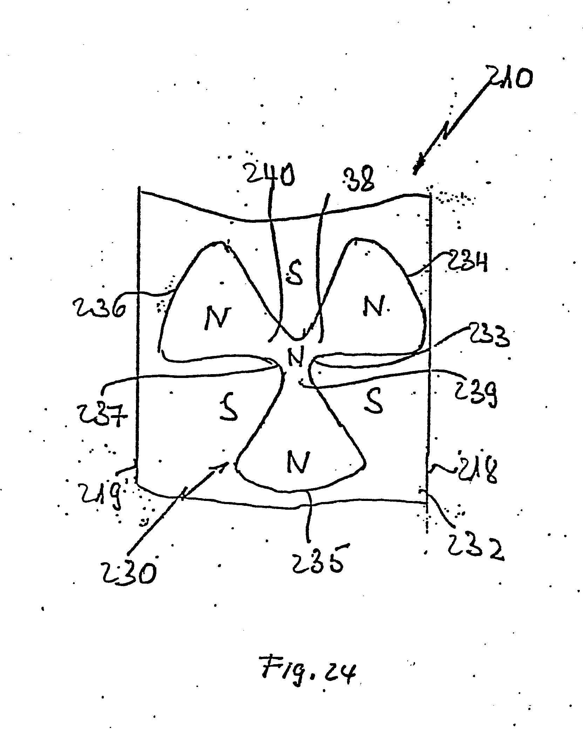

Die Darstellung der Fig. 24 zeigt die Magnetisierung des Folienstreifens (210) auf einem vorgegebenen Abschnitt (229), wobei die langen Kanten (218, 219) gezeichnet sind, während die Abbruchlinien im wesentlichen parallel zu den kurzen Kanten (227, 228) orientiert sind.

Die Umrißlinie (230) gibt die Grenzen eines Magnetpoles (231) wieder, welcher im Ausführungsbeispiel der Nordpol ist. Der Südpol (232) erstreckt sich jenseits der Umrißlinie (230) gemäß dem Ausführungsbeispiel der Südpole.

Wie sich aus der Darstellung der Fig. 24 ergibt, ist der Nordpol strahlenförmig um den Mittelpunkt (233) angeordnet. Hierüber sind drei um gleiche Bogenwinkel um den Mittelpunkt (233) versetzt angeordnete Strahlen (234-236) vorgesehen. Die Umrißlinie folgt damit einer Flügelschraube, deren Nabe (237) aus der Verbindung der Umrißlinien der drei Flügelwurzeln (238-240) der Schraubenflügel (234-236) entspricht.

Wie sich aus der Darstellung der Fig. 24 ergibt, nimmt die Umrißlinie der vorstehend beschriebenen Strahlenanordnung die volle Breite des betreffenden Folienabschnittes ein. Mehrere Strahlenanordnungen der beschriebenen Art sind nebeneinander über die Länge der Folienstreifen (210-213) vorgesehen.

**Ansprüche**

1. Medizinische Applikation zur Bekämpfung von Reizzuständen, insbesondere der Muskulatur, der Sehnen un der Gelenke, insbesondere von rheumatischen Beschwerden gekennzeichnet durch eine Bandage mit einseitig daran befestigten Bändern, die die Bandage der zu behandelnden Körperstelle fixieren und einen schichtweisen Aufbau aus einer Lage aus einem körpernahen Gewebe natürlicher Provinienz, einem darauf folgenden Kunstfasergewebe und einer Zwischenlage aus einem Thermovlies, welches aus einem offenzelligen Schaumstoff besteht, wobei in das Thermovlies Magnetfolienstreifen (4) eingebettet sind.

2. Bandage nach Anspruch 1, dadurch gekennzeichnet, daß die Magnetfolien (4) zwischen dem Thermovlies und dem aus Baumwolle bestehenden körpernahen Gewebe angeordnet sind.

3. Bandage nach einem der Ansprüche 1 oder 2, dadurch gekennzeichnet, daß das Kunstfasergewebe Brushedneylon ist.

4. Bandage nach einem oder mehreren der Ansprüche 1 bis 3, dadurch gekennzeichnet, daß die Bänder (3) elastisch sind.

5. Bandage nach einem oder mehreren der Ansprüche 1 bis 4, dadurch gekennzeichnet, daß die Bänder mit Hilfe von Klettverschlüssen (5) fixierbar sind.

6. Medizinische Applikation nach einem der Ansprüche 1 bis 5, dadurch gekennzeichnet, daß die Magnetfolie (23, 53, 54) wenigstens eine Zirkularmagnetisierung (41, 42, 49, 50) aufweist.

7. Medizinische Applikation nach einem der Ansprüche 1 bis 6, dadurch gekennzeichnet, daß in jeder Zirkularmagnetisierung (41, 42, 49, 50) die Magnete der Magnetfolie (23, 53, 54) im wesentlichen längs konzentrischer Ringe (27-30; 43-45) und zum geringen Teil längs bogenförmiger Strecken (34-37) angeordnet sind, welche jeweils benachbarte Ringe (27-30; 43-45) untereinander und alle Ringe (27-30; 43-45) von innen nach außen miteinander verbinden.

8. Medizinische Applikation nach einem der Ansprüche 1 bis 7, dadurch gekennzeichnet, daß mehrere Magnetfolien (23, 53, 54) einer Bandagenschicht nebeneinander angeordnet und teils rechteckigen bis quadratischen Umriß mit abgerundeten Ecken (47, 48), teils kreisscheibenförmigen Umriß (23) aufweisen, die eine konzentrische Magnetanordnung (39) aufweise, während die Magnete der Magnetfolien (53, 54) mit dem rechteckigen bis quadratischen Umriß in paarweise nebeneinander angeordneten, konzentrischen Ringen (41, 42, 49, 50) untergebracht sind.

9. Medizinische Applikation nach einem der Ansprüche 1 bis 3, dadurch gekennzeichnet, daß zwischen der Vliesschicht (25) und den Magnetfolien (23, 53, 54) eine Nickelfolie angeordnet ist.

10. Medizinische Applikation nach einem der Ansprüche 1 bis 9, dadurch gekennzeichnet, daß die zwischen Vliesschicht (25) und Magnetfolien (23, 53, 54) angeordnete Folie eine durchgehende Eisenfolie ist, die verzinnt ist.

11. Medizinische Applikation nach einem der Ansprüche 1 bis 10, gekennzeichnet durch einen Zuschnitt mit rechteckförmigem Umriß und einem Einschnitt (50) an einer längeren Rechteckseite, dessen Kanten vernäht sind, wobei rechteckförmige Magnetfolien (53, 54) längs der Einschnittkante und längs der längeren Kante (52) des rechteckförmigen Umrisses, sowie kreisscheibenförmige Magnetfolien (23) neben den kürzeren Rechteckseiten (51′) angeordnet sind.

12. Medizinische Applikation nach einem der Ansprüche 1 bis 11, dadurch gekennzeichnet, daß eine längere Rechteckseite (68) anstelle eines Einschnittes (50) eine angeschnittene Lasche (64) aufweist und in der Lasche eine kreisscheibenförmige Magnetfolie (23), sowie zwischen dieser und der an der längeren Rechteckseite (52) angeordneten Magnetfolie (53) eine weitere rechteckförmige Magnetfolie (54) angeordnet ist.

13. Medizinische Applikation nach einem der Ansprüche 1 bis 12, dadurch gekennzeichnet, daß die längeren Seiten (52, 68) des rechteckförmigen Bandagenzuschnittes (67) mit je einer angeschnittenen Lasche (64, 69) versehen und in jeder Lasche eine außenliegende, kreisscheibenförmige Magnetfolie (23) angeordnet ist, während rechteckförmige Magnetfolien (53) in Abständen nebeneinander vorgesehen sind, die mit ihren längeren Seiten parallel zur den kürzeren Rechteckseiten der Bandage (67) verlaufen.

14. Medizinische Applikation nach einem der Ansprüche 1 bis 13, dadurch gekennzeichnet, daß in jeder Seite des rechteckförmigen Zuschnittes der Bandage (74) Einschnitte (70-73) vorgesehen sind, die mit ihren Kanten vernäht sind, wobei in den Ecken (58, 59, 75, 76) der Bandage (74) kreisscheibenförmige Magnetfolien (23) und parallel zu den längeren Seiten (28, 52, 58) der Bandage (74) rechteckförmige Magnetfolien (53) vorgesehen sind.

15. Medizinische Applikation nach einem der Ansprüche 1 bis 14, dadurch gekennzeichnet, daß die rechteckförmigen Magnetfolien (53, 54) mit jeweils zwei nebeneinander angeordneten Zirkularmagnetisierungen (40, 41; 49, 50) versehen sind.

16. Medizinische Applikation nach einem der Ansprüche 1 bis 15, dadurch gekennzeichnet, daß die Ringe der Zirkularmagnetisierungen (41, 42, 49, 50) mit innenliegenden Vollringen (20, 19) und außenliegenden Ringsegmenten (27, 43) ausgeführt sind.

17. Medizinische Applikation gegen Migränezustände, insbesondere nach einem der Ansprüche 1 bis 16, gekennzeichnet durch ein Stirnband aus einem rechteckigen Streifen einer flexiblen, axialmagnetisierten Magnetfolie (102) mit einem Stoffüberzug (103), dessen beide Enden (104, 105) durch ein längenveränderliches Band (106) miteinander verbunden sind.

18. Medizinische Applikation nach Anspruch 17, dadurch gekennzeichnet, daß das längenveränderliche Band (106) aus einem elastischen Gewebe besteht.

19. Medizinische Applikation nach den Ansprüchen 17 oder 18, dadurch gekennzeichnet, daß der Stoffüberzug (103) ein Frotteegewebe aus Natur-Ringgarnen ist.

20. Medizinische Applikation nach einem oder mehreren der Ansprüche 17 bis 19, dadurch gekennzeichnet, daß der Magnetstreifen zumindest stellenweise zirkularmagnetisiert ist.

21. Medizinische Applikation gekennzeichnet durch eine Decke, insbesondere zur Verwendung als Unterbett, deren Liegefläche aus Schafwolle (115) besteht und einer Schicht aus Baumwollgewebe (112) eine Schicht aus Polyester-Vlies (113) vorgesehen ist, welches aus einem offenzelligen Schaumstoff gebildet ist, in dem senkrecht zur Deckenlängsachse und parallel zueinander folienförmige, flexible Magnetelemente (116) eingebettet sind.

22. Medizinische Applikation nach Anspruch 21, dadurch gekennzeichnet, daß die den Südpol bildende Oberfläche der Magnetelemente (116) am Fußende (118) und die den Nordpol bildende Oberfläche der Magnetelemente (116) am Kopfende (117) in der zur Polyester-Vliesschicht (113) weist.

23. Medizinische Applikation nach Anspruch 21 oder 22, dadurch gekennzeichnet, daß die kopfendseitigen Magnetelemente (116) in der Nähe der Deckenlängsachse und zwar symmetrisch zu derselben eine Zirkularmagnetisierung (129, 143, 144) aufweisen.

24. Medizinische Applikation nach einem der Ansprüche 21 bis 23, dadurch gekennzeichnet, daß die Magnetelemente (116) in der Grenzschicht des Polyester-Vlieses (113) zur Schlafwollage (115) angeordnet sind.

25. Medizinische Applikation nach einem oder mehreren der Ansprüche 21 bis 24, dadurch gekennzeichnet, daß die Oberfläche der Polyester-Vliesschicht (113) mit einer dünnen Harzschicht (114) versehen ist.

26. Medizinische Applikation nach den Ansprüchen 21 bis 25, dadurch gekennzeichnet, daß das Polyester-Vlies (113) aerodynamisch geblasen ist.

27. Veterinärmedizinische Applikation zur Wärmebehandlung isolierter Körperpartien mit Hilfe eines Umschlages, der als wärmewirksamer Bestandteil wenigstens ein Flachkissen mit der zu bandagierenden Körperpartie und/oder der Bandage angepaßten Umriß aufweist, bei dem der Kissenbezug aus insbesondere Baumwollstoffabschnitten besteht, die an ihren Rändern miteinander verbunden sind und die Kissenfüllung aus der Doppellage eines Vlieses vom Umriß der Stoffabschnitte (203, 204), sowie aus Magnetstreifen besteht, welche quer zur Wickelrichtung des Umschlages verlaufen und an der körpernahen Kissenbezugseite angeordnet sind, dadurch gekennzeichnet, daß die Magnetstreifen (210-213) einer Streifenrichtung aus einem magnetisierten Folienabschnitt bestehen, der mit einer Seite auf dem Stoffausschnitt (204) und mit der anderen Seite auf dem darunterliegenden Vlies (209) aufliegt, und daß gleichnamige Magnetpole (231) der Magnetisierung in Strahlen (234-236) um einen Mittelpunkt (233) angeordnet sind.

28. Veterinärmedizinische Applikation nach Anspruch 27, dadurch gekennzeichnet, daß die Strahlenanordnung (230) der gleichnamigen Magnetpole dem Umriß einer Flügelschraube folgt, deren Nabe (237) die ineinander übergehenden Umrißlinien der Schraubenflügelwurzeln (238-240) bilden.

29. Veterinärmedizinische Applikation nach einem der Ansprüche 27 oder 28, dadurch gekennzeichnet, daß die Strahlenanordnung (230) der gleichnamigen Magnetpole dem Umriß einer dreiflügeligen Schraube (233-236) folgt.

30. Veterinärmedizinische Applikation nach einem der Ansprüche 27 bis 29, dadurch gekennzeichnet, daß die Strahlenanordnung (230) die volle Breite der im Grundriß (218, 219, 227, 228) rechteckigen Folienabschnitte (210-213) aufweist und mehrere Strahlenanordnungen (230) nebeneinander über die Länge der Folienstreifen (210-213) vorgesehen sind, die im wesentlichen ebenso groß wie die Kissenbreite quer zur Wickelrichtung der Bandage ist.

31. Veterinärmedizinische Anordnung nach einem der Ansprüche 27 bis 30, dadurch gekennzeichnet, daß jeder der mit quer zur Wickelrichtung der Bandage abgesteppten Kissenabschnitte (222-224) Magnetstreifen (210-213) aufweist, deren Längskanten (218, 219) parallel zu den Steppnähten (225, 226) des Kissens (201) verlaufen.

32. Veterinärmedizinische Applikation nach einem der Ansprüche 27 bis 31, dadurch gekennzeichnet, daß die parallel zu den Steppnähten (225, 226) angeordneten Folienstreifen in den außen liegenden Kissenabschnitten (222, 224) so angeordnet sind, daß ihre Längsmitte etwa mit der Mitte des betreffenden Kissenabschnittes (222, 224) fluchtet, während im Mittelabschnitt (223) des Kissens (201) angeordnete Magnetstreifen (211, 212) symmetrisch zu dessen Längsmitte angeordnet sind.

Fig. 1

0 283 873

Fig. 1a

Fig. 2

Fig. 3

0 283 873

Fig. 4

Fig. 5

Fig. 6

Fig. 7

Fig. 8

Fig. 9

Fig. 10

0 283 873

Fig. 11

0 283 873

Fig. 12

Fig. 13

Fig. 14

0 283 873

Fig.16

114  116  115  116  111

**Fig.16**

113  114  112

117

N

N

33  33

116

111

S

S

116

118

**Fig. 17**

Fig. 18

0 283 873

Fig. 19

Fig. 20

Fig. 21

Fig. 22

Fig. 23

0 283 873

Fig. 24